# EUROPEAN PATENT APPLICATION

(11) **EP 4 678 640 A1**
(43) Date of publication of application: **14.01.2026**
(21) Application number: 24769780.8
(22) Date of filing: 04.03.2024
(51) Int. Cl.: C07D 405/14, C07D 401/14, C07D 413/14, C07D 471/04, C07D 491/044, C07D 491/048, C07D 498/04, A61K 31/41, A61K 31/63, A61P 35/00

(54) **AROMATIC AMIDE DERIVATIVE, AND PREPARATION METHOD THEREFOR AND USE THEREOF**

(30) Priority: 10.03.2023 CN 202310228489; 03.11.2023 CN 202311453613
(71) Applicant: Zhejiang Hisun Pharmaceutical Co., Ltd., Taizhou, Zhejiang 318000 (CN); Shanghai Aryl Pharmtech Co., Ltd., Songjiang, Shanghai 201612 (CN)
(72) Inventor: QIU, Zongxing, Shanghai 201612 (CN); ZHANG, Panpan, Zhejiang 318000 (CN); PENG, Jilei, Shanghai 201612 (CN); CAO, Hai, Shanghai 201612 (CN); WANG, Zefeng, Shanghai 201612 (CN); SHI, Yaqiong, Shanghai 201612 (CN); YE, Cheng, Shanghai 201612 (CN); GUO, Ze, Zhejiang 318000 (CN); XU, Daiwang, Shanghai 201612 (CN); XU, Xiaojie, Zhejiang 318000 (CN)
(74) Representative: Kador & Partner Part mbB
(86) International application number: PCT/CN2024/079907
(87) International publication number: WO 2024/188086

(57) **Abstract**

The present invention relates to an aromatic amide derivative, a preparation method therefor, and use of a pharmaceutical composition containing the derivative in medicine. Specifically, the invention relates to an aromatic amide derivative as shown by general formula (I), a preparation method therefor, a pharmaceutically acceptable salt thereof, and a use thereof as a therapeutic agent, in particular a KIF18A inhibitor, wherein the definition of each substituent in general formula (I) is the same as the definition in the description,

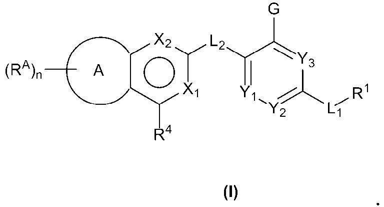

## Description

### TECHNICAL FIELD

The present invention relates to an aromatic amide derivative, a preparation method therefor, a pharmaceutical composition containing the derivative and use thereof as a therapeutic agent, particularly as a KIF18A inhibitor.

### BACKGROUND

Kinesin molecule is a motor protein with a microtubule as an orbit, and plays an important role in organelle migration, tissue and organ development, signal transduction, mitosis, meiosis, and other processes. Multiple microtubule-associated proteins (MAPs) of kinesin-8 family play a role in regulating the dynamic instability of microtubule by influencing the polymerization and depolymerization of microtubule. KIF18A is a member of kinesin-8 family, can move towards a positive pole with the microtubule as the orbit, and tends to bind to a longer microtubule, its activity is length-dependent and affects a spindle body length, which can ensure the timely and smooth completion of sister chromosome array, and the KIF18A has very similar functions in different species, thus being conservative.

KIF18A is a molecular motor protein moving towards a positive pole end of a microtubule with the microtubule as an orbit, regulates chromosome congression by influencing the dynamic instability of microtubule end, and plays a role in a mitosis phase. In a mitosis anaphase, this protein is ubiquitinated and degraded, which ensures the accurate chromosome separation during mitosis, and promotes the smooth completion of mitosis and cytokinesis. In a mitosis prophase, locating the KIF18A at a positive pole end of a microtubule close to a kinetochore is a necessary condition to exert its function, the localization depends not only on a motor activity of its N-terminal, but also on a tail structural domain with a microtubule binding ability. The KIF18A is also modified by reversible protein phosphorylation/dephosphorylation, but there is still a lack of systematic study on how the post-translational modification of this protein regulates the function of the KIF18A. An estrogen receptor ERα may bind to the KIF18A and promote its transcription, but it is still unclear whether the KIF18A is regulated by other transcription factors, so that a gene transcription regulation mechanism of the KIF18A needs to be further studied. In a meiosis process, cells without the KIF18A will be unable to complete meiosis, which will lead to dyszoospermia and testicular dysgenesis of male animals.

Studies show that KIF18A protein is highly expressed in many cancers, comprising, but not limited to, a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, and the like, which indicates that the KIF18A is closely related to the occurrence and development of tumors, and may become a target for molecular diagnosis and treatment of various tumors. The expression of the KIF18A is related to the development of clinical colorectal cancer. Studies show that the KIF18A can induce Akt phosphorylation, knocking out the KIF18A in mice can obviously promote cell apoptosis, and it is supposed that the KIF18A can promote the occurrence and development of colorectal cancer by activating a PI3K-Akt signaling pathway. The KIF18A is also highly expressed in human breast cancer cells, and its overexpression is related to the classification, migration and prognosis of breast tumor. Studies on the breast cancer show that the overexpression of KIF18A may lead to the generation of multinucleated cells, while the low expression of KIF18A can greatly weaken a cell proliferation ability in vitro and in vivo, which is due to cell apoptosis induced by microtubule stabilization at the microtubule end through KIF18A and the inactivation of PI3K-Akt signal transduction pathway. In addition, KIF18A is up-regulated in transcription and translation levels in a lung adenocarcinoma, and the abnormal expression of KIF18A is related to a clinicopathological malignancy grade. KIF18 gene mutation can be observed in the lung adenocarcinoma, its expression is also regulated by a DNA copy number, and KIF18A gene knockout can inhibit the proliferation of lung adenocarcinoma cells in vivo and in vitro, thus inducing cell apoptosis and G2/M phase arrest. Genes highly expressed together with the KIF18A are all concentrated in a cell cycle signaling pathway, so that it is of great clinical significance to further study an action mechanism of KIF18A in tumors.

There is no new drug on the market for inhibitors targeting KIF18A, and at present, only AMG-650 from Amgen Company of the United States has entered a clinical phase I. As a relatively advanced research direction, there is still a huge exploration space for correlational studies on a KIF18A target, so that it is very necessary to continuously study its action mechanism and develop new inhibitors.

### SUMMARY

Aiming at the above technical problems, the present invention provides an aromatic amide derivative as shown by general formula (I), or a stereoisomer thereof, a tautomer thereof or a pharmaceutically acceptable salt thereof: 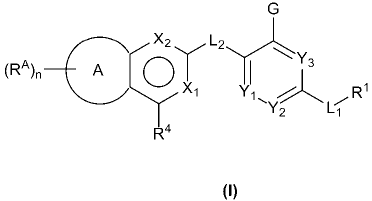 wherein:
ring A is selected from 5-7 membered heterocyclyl or 5-7 membered heteroaryl;
G is selected from 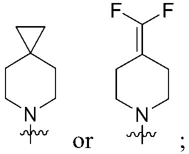
X₁ and X₂ are each independently selected from CR^{a} or an N atom;
Y₁, Y₂ and Y₃ are each independently selected from CR^{b} or an N atom, and at most two of Y₁, Y₂ and Y₃ are N atom at the same time;
R^{a} and R⁶ are each independently selected from a hydrogen atom, halogen, hydroxyl, cyano, alkyl or alkoxy, wherein the alkyl or the alkoxy is optionally further substituted by one or more substituents selected from halogen, hydroxyl, cyano, alkyl or alkoxy;
R^{A} is the same or different, and is each independently selected from a hydrogen atom, halogen, hydroxyl, cyano, alkyl or alkoxy, wherein the alkyl or the alkoxy is optionally further substituted by one or more substituents selected from halogen, hydroxyl, cyano, alkyl or alkoxy;
or, two R^{A} form a C(O) together with the same carbon atom to which the two R^{A} are attached;
L₁ is selected from a bond or C₁-C₆ alkylene, wherein the alkylene is optionally further substituted by one or more substituents selected from halogen, hydroxyl, cyano or alkoxy, and one or more methylene groups of the alkylene are optionally substituted by one or more O, S(O)ᵣ, C(O) or NR^{c};
R^{c} is selected from a hydrogen atom or alkyl;
L₂ is selected from 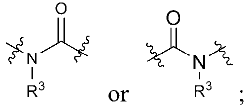
R³ is each independently selected from a hydrogen atom or alkyl, wherein the alkyl is optionally further substituted by one or more substituents selected from halogen, hydroxyl, cyano or alkoxy; and R³ is preferably a hydrogen atom;
R¹ is selected from a hydrogen atom, cyano, halogen, alkyl, hydroxyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁴ is selected from cyano, halogen, alkyl, alkenyl, alkynyl, hydroxyl, cycloalkyl, heterocyclyl, aryl, heteroaryl, -OR⁵, -C(O)R⁵, -C(O)OR⁵, -NHC(O)R⁵, -NHC(O)OR⁵, -NR⁶R⁷, -C(O)NR⁶R⁷, -CH₂NHC(O)OR⁵, -CH₂NR⁶R⁷ or -S(O)ᵣR⁵, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁵ is each independently selected from a hydrogen atom, alkyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁶ and R⁷ are each independently selected from a hydrogen atom, hydroxyl, halogen, alkyl, alkoxy, cycloalkyl, heterocyclyl, aryl or heteroaryl, wherein the alkyl, the alkoxy, the cycloalkyl, the heterocyclyl, the aryl or the heteroaryl is optionally further substituted by one or more substituents selected from hydroxyl, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
or, R⁶ and R⁷ form a 4-8 membered heterocyclyl together with atoms to which the R⁶ and the R⁷ are attached, wherein the 4-8 membered heterocyclyl contains one or more N, O or S(O)r, and the 4-8 membered heterocyclyl is optionally further substituted by one or more substituents selected from hydroxyl, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁸, R⁹ and R¹⁰ are each independently selected from a hydrogen atom, alkyl, amino, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or carboxylate group.
n is 0, 1, 2, 3 or 4; and
r is each independently 0, 1 or 2.

A preferred solution of the present invention provides a compound as shown by general formula (I), or a stereoisomer thereof, a tautomer thereof or a pharmaceutically acceptable salt thereof, which is a compound as shown by formula (II) or (III), or a stereoisomer thereof, a tautomer thereof or a pharmaceutically acceptable salt thereof, wherein: 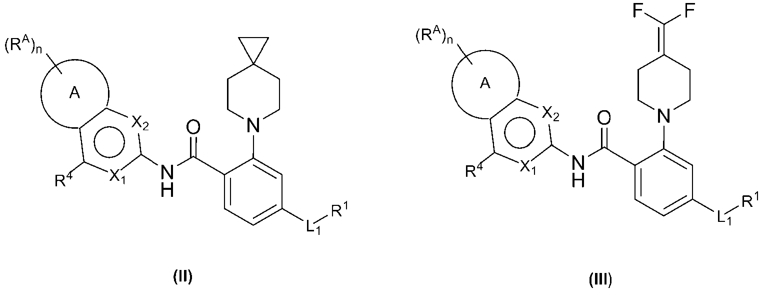 definitions of ring A, X₁, X₂, R^{A}, R¹, R⁴, L₁ and n are as described in the general formula (I).

A preferred solution of the present invention provides a compound as shown by general formula (I), (II) or (III), or a stereoisomer thereof, a tautomer thereof or a pharmaceutically acceptable salt thereof, wherein:
L₁ is selected from a bond or C₁-C₆ alkylene, wherein the alkylene is optionally further substituted by one or more hydroxyl groups, and one or more methylene groups of the alkylene are optionally substituted by one or more O, S(O)ᵣ, C(O) or NR^{c};
r is 2; and
R^{c} is selected from a hydrogen atom or methyl.
A preferred solution of the present invention provides a compound as shown by general formula (I), (II) or (III), or a stereoisomer thereof, a tautomer thereof or a pharmaceutically acceptable salt thereof, wherein L₁ is selected from a bond, -NHSO₂CH₂CH₂-, -SO₂NHCH₂CH₂-, -SO₂-, -CH₂SO₂-, -NHSO₂-, -SO₂NH-, -NHC(CH₃)₂CH₂-, -C(O)NHCH₂CH₂-, -C(O)NHC(CH₃)₂CH₂-, -C(O)N(CH₃)CH₂CH₂-, -CH(CH₃)(OH)CH₂-, -NHSO₂CH(CH₃)CH₂-, -SO₂NHC(CH₃)₂CH₂-, -C(O)NH-, -NHCH₂CH₂- or -CH₂SO₂CH₂CH₂-.
A preferred solution of the present invention provides a compound as shown by general formula (I), (II) or (III), or a stereoisomer thereof, a tautomer thereof or a pharmaceutically acceptable salt thereof, wherein R¹ is selected from a hydrogen atom, hydroxyl, alkyl, heterocyclyl, cycloalkyl or heteroaryl, wherein the alkyl, the heterocyclyl, the cycloalkyl or the heteroaryl is optionally further substituted by one or more substituents selected from hydroxyl or alkyl.

A preferred solution of the present invention provides a compound as shown by general formula (I), (II) or (III), or a stereoisomer thereof, a tautomer thereof or a pharmaceutically acceptable salt thereof, wherein 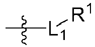 is

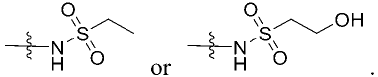

A preferred solution of the present invention provides a compound as shown by general formula (I), (II) or (III), or a stereoisomer thereof, a tautomer thereof or a pharmaceutically acceptable salt thereof, wherein 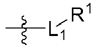 is

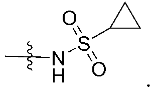

A preferred solution of the present invention provides a compound as shown by general formula (I), (II) or (III), or a stereoisomer thereof, a tautomer thereof or a pharmaceutically acceptable salt thereof, wherein X₁ and X₂ are each independently selected from CH.

A preferred solution of the present invention provides a compound as shown by general formula (I), (II) or (III), or a stereoisomer thereof, a tautomer thereof or a pharmaceutically acceptable salt thereof, wherein ring A is selected from:

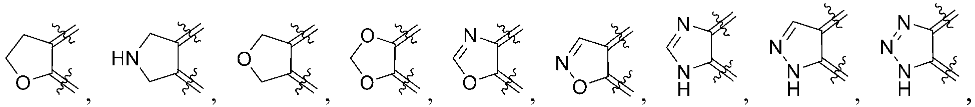

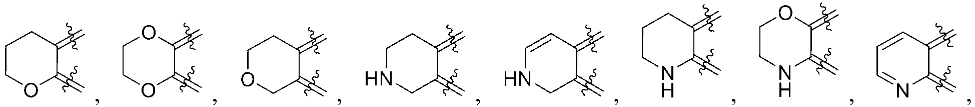

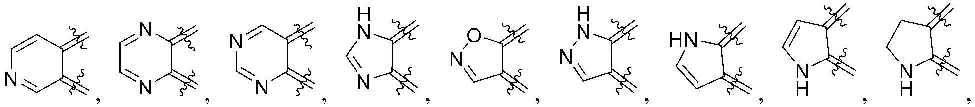

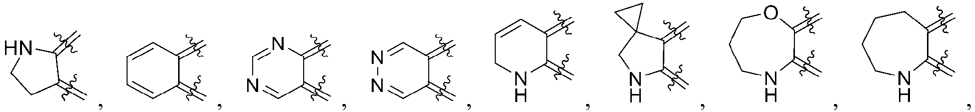

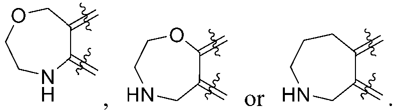

A preferred solution of the present invention provides a compound as shown by general formula (I), (II) or (III), or a stereoisomer thereof, a tautomer thereof or a pharmaceutically acceptable salt thereof, wherein ring A is selected from 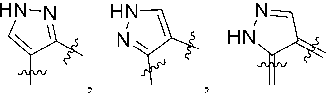 or

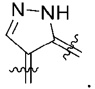

A preferred solution of the present invention provides a compound as shown by general formula (I), (II) or (III), or a stereoisomer thereof, a tautomer thereof or a pharmaceutically acceptable salt thereof, wherein:
R^{A} is each independently selected from a hydrogen atom or methyl;
or, two R^{A} form a C(O) together with the same carbon atom to which the two R^{A} are attached.

A preferred solution of the present invention provides a compound as shown by general formula (I), (II) or (III), or a stereoisomer thereof, a tautomer thereof or a pharmaceutically acceptable salt thereof, wherein:
R⁴ is each independently selected from 3-10 membered heterocyclyl or C₃-C₁₀ cycloalkyl, wherein the heterocyclyl or the cycloalkyl is optionally further substituted by one or more hydroxyl groups or halogens.

A preferred solution of the present invention provides a compound as shown by general formula (I), (II) or (III), or a stereoisomer thereof, a tautomer thereof or a pharmaceutically acceptable salt thereof, wherein:
R⁴ is each independently selected from C₂-C₆ alkenyl or C₁-C₆ alkyl, wherein the alkenyl or the alkyl is optionally further substituted by one or more hydroxyl groups or halogens.

A preferred solution of the present invention provides a compound as shown by general formula (I), (II) or (III), or a stereoisomer thereof, a tautomer thereof or a pharmaceutically acceptable salt thereof, wherein:
R⁴ is

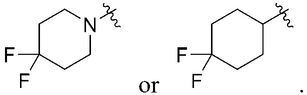

A preferred solution of the present invention provides a compound as shown by general formula (I), (II) or (III), or a stereoisomer thereof, a tautomer thereof or a pharmaceutically acceptable salt thereof, wherein R⁴ is

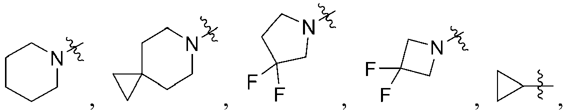

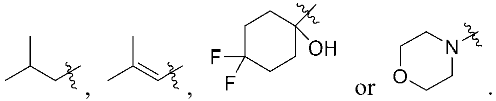

In a preferred solution of the present invention, the compound as shown by general formula (I), (II) or (III) is selected from: 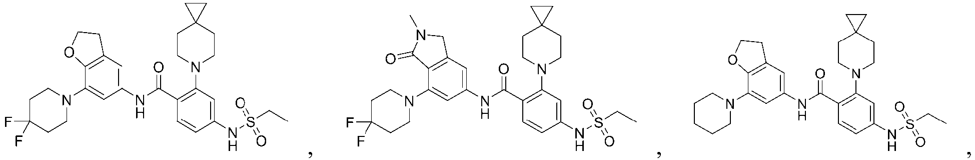 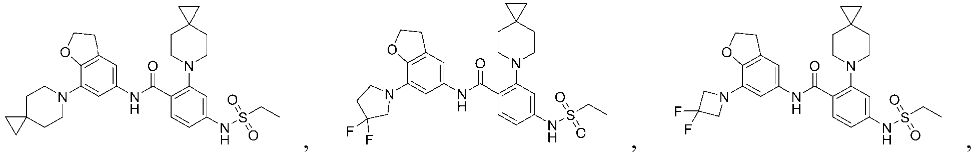 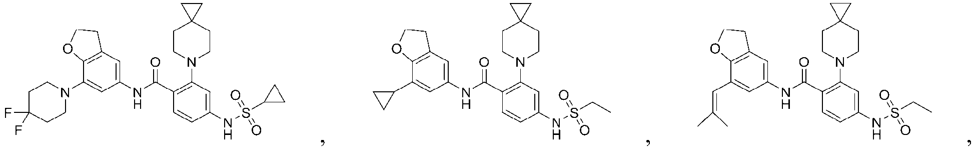 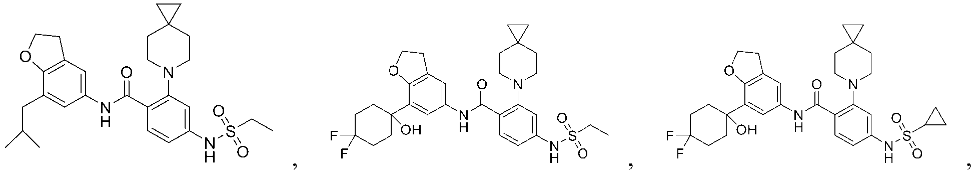 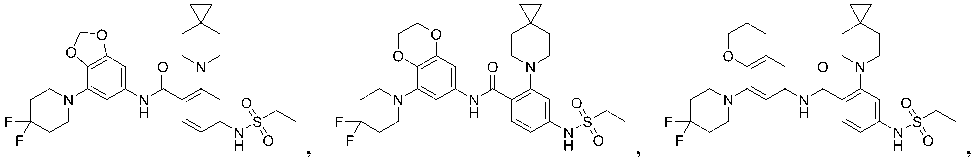 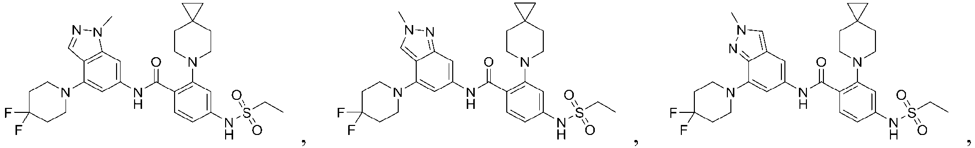 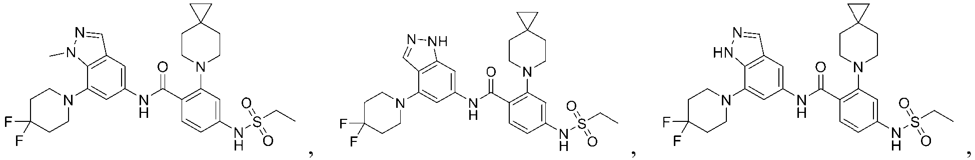 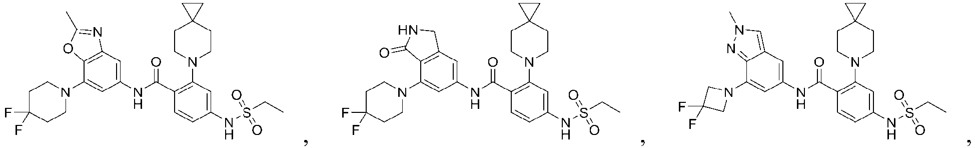 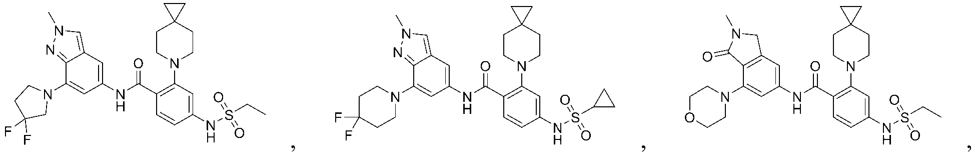 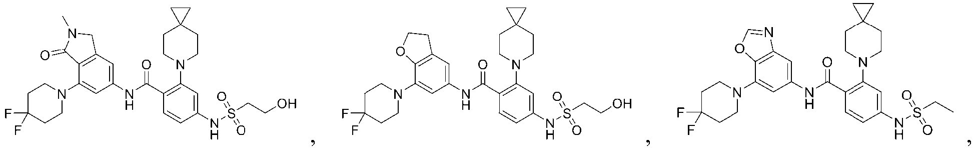 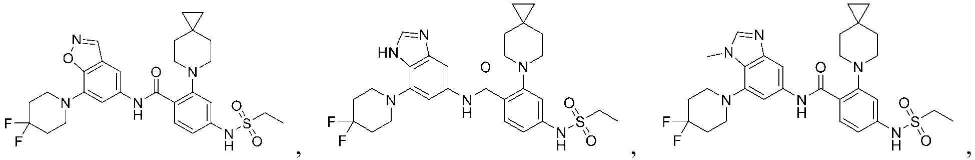 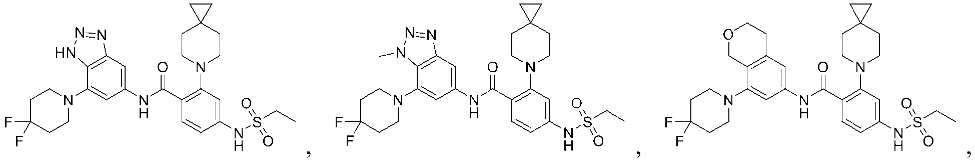 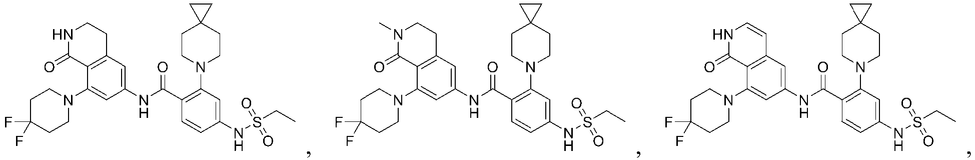 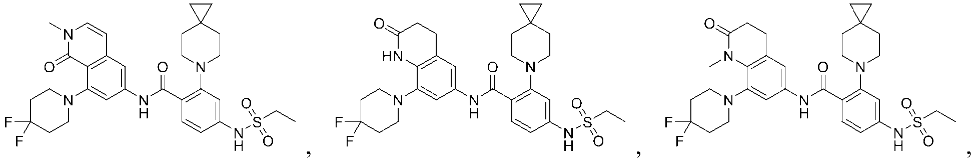 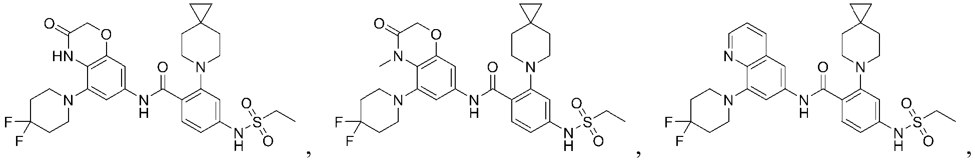 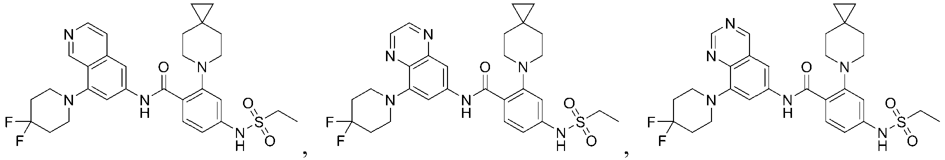 or

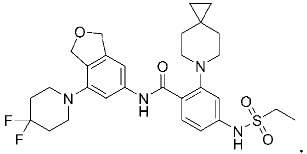

In a further preferred solution of the present invention, the compound as shown by general formula (I), (II) or (III) is selected from:

| Serial number of compound | Structure | Name |
|---|---|---|
| Example 1 | 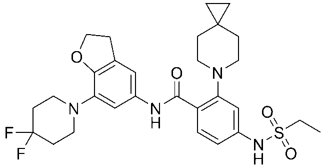 | N-(7-(4,4-difluoropiperidin-1-yl)-2,3-dihydro benzofuran-5-yl)-4-(ethylsulfonamido)-2-(6-a zaspiro[2.5]octan-6-yl)benzamide |
| Example 2 | 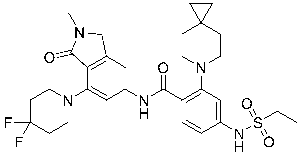 | N-(7-(4,4-difluoropiperidin-1-yl)-2-methyl-1-oxoisoindolin-5-yl)-4-(ethylsulfonamido)-2-( 6-azaspiro[2.5]octan-6-yl)benzamide |
| Example 3 | 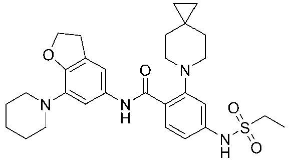 | 4-(ethylsulfonamido)-N-(7-(piperidin-1-yl)-2, 3-dihydrobenzofuran-5-yl)-2-(6-azaspiro[2.5] octan-6-yl)benzamide |
| Example 4 | 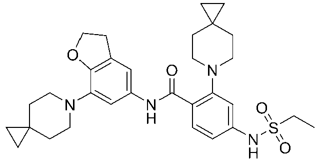 | N-(7-(6-azaspiro[2.5]octan-6-yl)-2,3-dihydro benzofuran-5-yl)-4-(ethylsulfonamido)-2-(6-a zaspiro[2.5]octan-6-yl)benzamide |
| Example 5 | 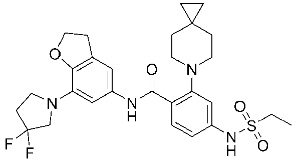 | N-(7-(3,3-difluoropyrrolidine-1-yl)-2,3-dihyd robenzofuran-5-yl)-4-(ethylsulfonamido)-2-(6 -azaspiro[2.5]octan-6-yl)benzamide |
| Example 6 | 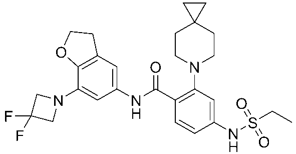 | N-(7-(3,3-difluoroazetidine-1-yl)-2,3-dihydro benzofuran-5-yl)-4-(ethylsulfonamido)-2-(6-a zaspiro[2.5]octan-6-yl)benzamide |
| Example 7 | 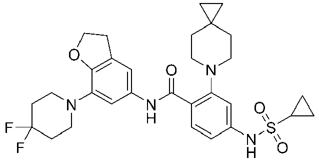 | 4-(cyclopropanesulfonamido)-N-(7-(4,4-diflu oropyridin-1-yl)-2,3-dihydrobenzofuran-5-yl) -2-(6-azaspiro[2.5]octan-6-yl)benzamide |
| Example 8 | 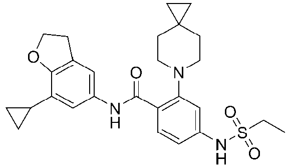 | N-(7-cyclopropyl-2,3-dihydrobenzofuran-5-yl )-4-(ethylsulfonamido)-2-(6-azaspiro[2.5]octa n-6-yl)benzamide |
| Example 9 | 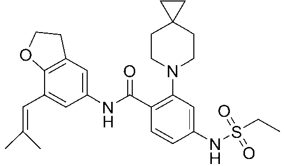 | 4-(ethylsulfonamido)-N-(7-(2-methylprop-1-e n-1-yl)-2,3-dihydrobenzofuran-5-yl)-2-(6-aza spiro[2.5]octan-6-yl)benzamide |
| Example 10 | 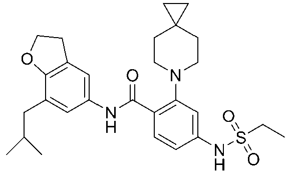 | 4-(ethylsulfonamido)-N-(7-isobutyl-2,3-dihyd robenzofuran-5-yl)-2-(6-azaspiro[2.5]octan-6 -yl)benzamide |
| Example 11 | 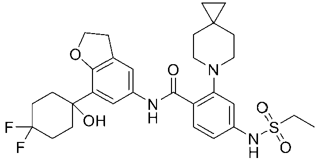 | N-(7-(4,4-difluoro-1-hydroxycyclohexyl)-2,3-dihydrobenzofuran-5-yl)-4-(ethylsulfonamido )-2-(6-azaspiro[2.5]octan-6-yl)benzamide |
| Example 12 | 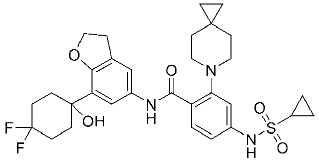 | 4-(cyclopropanesulfonamido)-N-(7-(4,4-diflu oro-1-hydroxycyclohexyl)-2,3-dihydrobenzof uran-5-yl)-2-(6-azaspiro[2.5]octan-6-yl)benza mide |
| Example 13 | 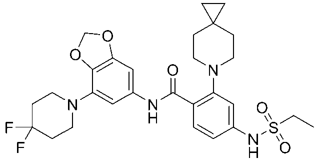 | N-(7-(4,4-difluoropiperidin-1-yl)benzo[d][1,3 ]dioxol-5-yl)-4-(ethylsulfonamido)-2-(6-azas piro[2.5]octan-6-yl)benzamide |
| Example 14 | 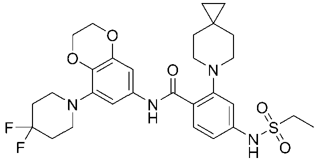 | N-(8-(4,4-difluoropiperidin-1-yl)-2,3-dihydro benzo[b][1,4]dioxin-6-yl)-4-(ethylsulfonamid o)-2-(6-azaspiro[2.5]octan-6-yl)benzamide |
| Example 15 | 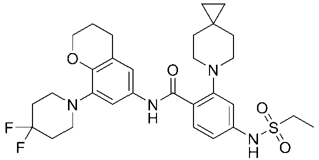 | N-(8-(4,4-difluoropiperidin-1-yl)dihydrobenz ofuran-6-yl)-4-(ethylsulfonamido)-2-(6-azasp iro[2.5]octan-6-yl)benzamide |
| Example 16 | 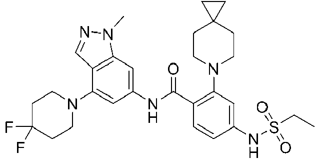 | N-(4-(4,4-difluoropiperidin-1-yl)-1-methyl-1 H-indazol-6-yl)-4-(ethylsulfonamido)-2-(6-az aspiro[2.5]octan-6-yl)benzamide |
| Example 17 | 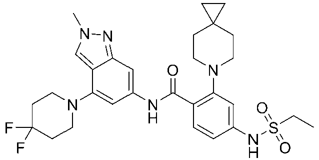 | N-(4-(4,4-difluoropiperidin-1-yl)-2-methyl-2 H-indazol-6-yl)-4-(ethylsulfonamido)-2-(6-az aspiro[2.5]octan-6-yl)benzamide |
| Example 18 | 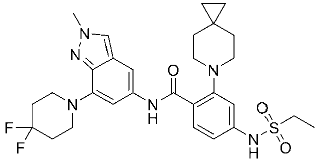 | N-(7-(4,4-difluoropiperidin-1-yl)-2-methyl-2 H-indazol-5-yl)-4-(ethylsulfonamido)-2-(6-az aspiro[2.5]octan-6-yl)benzamide |
| Example 19 | 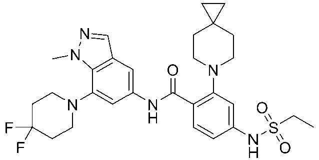 | N-(7-(4,4-difluoropiperidin-1-yl)-1-methyl-1 H-indazol-5-yl)-4-(ethylsulfonamido)-2-(6-az aspiro[2.5]octan-6-yl)benzamide |
| Example 20 | 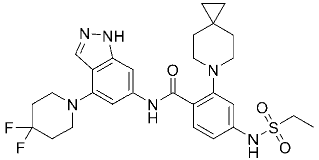 | N-(4-(4,4-difluoropiperidin-1-yl)-1H-indazol-6-yl)-4-(ethylsulfonamido)-2-(6-azaspiro[2.5] octan-6-yl)benzamide |
| Example 21 | 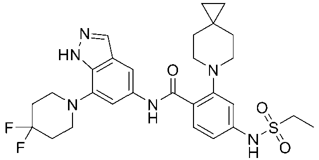 | N-(7-(4,4-difluoropiperidin-1-yl)-1H-indazol-5-yl)-4-(ethylsulfonamido)-2-(6-azaspiro[2.5] octan-6-yl)benzamide |
| Example 22 | 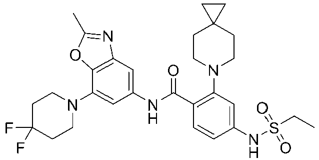 | N-(7-(4,4-difluoropiperidin-1-yl)-2-methylbe nzo[d]oxazol-5-yl)-4-(ethylsulfonamido)-2-(6 -azaspiro[2.5]octan-6-yl)benzamide |
| Example 23 | 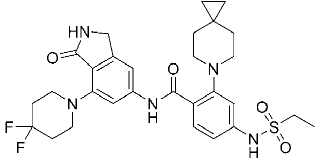 | N-(7-(4,4-difluoropiperidin-1-yl)-1-oxoisoind olin-5-yl)-4-(ethylsulfonamido)-2-(6-azaspiro [2.5]octan-6-yl)benzamide |
| Example 24 | 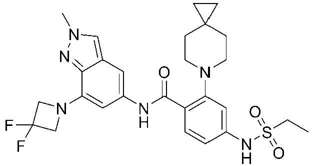 | N-(7-(3,3-difluoroazetidin-1-yl)-2-methyl-2H -indazol-5-yl)-4-(ethylsulfonamido)-2-(6-azas piro[2.5]octan-6-yl)benzamide |
| Example 25 | 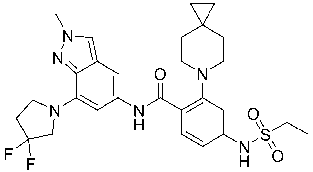 | N-(7-(3,3-difluoropyrrolidin-1-yl)-2-methyl-2 H-indazol-5-yl)-4-(ethylsulfonamido)-2-(6-az aspiro[2.5]octan-6-yl)benzamide |
| Example 26 | 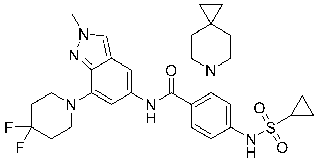 | 4-(cyclopropanesulfonamido)-N-(7-(4,4-diflu oropyridin-1-yl)-2-methyl-2H-indazol-5-yl)-2 -(6-azaspiro[2.5]octan-6-yl)benzamide |
| Example 27 | 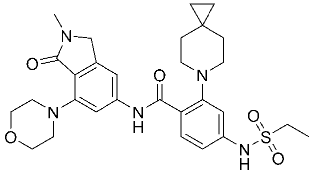 | 4-(ethylsulfonamido)-N-(2-methyl-7-morphol ino-1-oxoisoindolin-5-yl)-2-(6-azaspiro[2.5]o ctan-6-yl)benzamide |
| Example 28 | 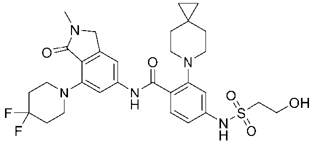 | N-(7-(4,4-difluoropiperidin-1-yl)-2-methyl-1-oxoisoindolin-5-yl)-4-((2-hydroxyethyl)sulfa mine)-2-(6-azaspiro[2.5]octan-6-yl)benzamid e |
| Example 29 | 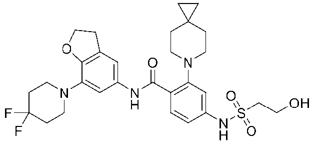 | N-(7-(4,4-difluoropiperidin-1-yl)-2,3-dihydro benzofuran-5-yl)-4-((2-hydroxyethyl)sulfami ne)-2-(6-azaspiro[2.5]octan-6-yl)benzamide |

or the stereoisomer thereof, the tautomer thereof or the pharmaceutically acceptable salt thereof.

Note: if there is a difference between the drawn structure and the given name of the structure, the drawn structure will be given greater weight.

Further, the present invention provides a pharmaceutical composition, wherein the pharmaceutical composition contains an effective amount of the compound as shown by general formula (I), (II) or (III), or the stereoisomer thereof, the tautomer thereof or the pharmaceutically acceptable salt thereof, and a pharmaceutically acceptable carrier, an excipient or a combination of the two.

The present invention provides use of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) as a medicament (such as use for treatment).

The present invention provides use of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) in the preparation of a KIF18A inhibitor.

The present invention further provides use of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) in the preparation of a medicament for treating a KIF18A-mediated disease, wherein the KIF18A-mediated disease is preferably a cancer; wherein the KIF18A-mediated disease is more preferably selected from a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, a colorectal cancer and a lung adenocarcinoma.

The present invention further provides use of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) in the preparation of a medicament for treating cancer.

The present invention provides use of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) in the preparation of a medicament for treating a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, a colorectal cancer and a lung adenocarcinoma.

The present invention further provides a method for preventing and/or treating a KIF18A-mediated disease, which comprises administering an effective amount of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) to a patient; wherein the KIF18A-mediated disease is preferably cancer; wherein the KIF18A-mediated disease is more preferably selected from a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, a colorectal cancer and a lung adenocarcinoma.

The present invention further provides a method for preventing and/or treating cancer, which comprises administering an effective amount of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) to a patient.

The present invention further provides a method for preventing and/or treating a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, a colorectal cancer or a lung adenocarcinoma, which comprises administering an effective amount of the compound as shown by general formula (I), (II) or (III), or the stereoisomer thereof, the tautomer thereof or the pharmaceutically acceptable salt thereof, or the pharmaceutical composition thereof (such as the pharmaceutical composition described in the above technical solution) to a patient.

### DETAILED DESCRIPTION

Unless stated to the contrary, some terms used in the specification and the claims of the present invention are defined as follows.

"Bond" refers to that a labeled substituent does not exist, and two end parts of the substituent are directly connected to form the bond.

"Alkyl" refers to an aliphatic hydrocarbon group comprising a C₁-C₂₀ straight chain or branched chain when taken as one group or a part of one group. Preferably, the alkyl is C₁-C₁₀ alkyl, and more preferably, the alkyl is C₁-C₆ alkyl. Embodiments of an alkyl group comprise, but are not limited to, methyl, ethyl, n-propyl, isopropyl, n-butyl, isobutyl, tert-butyl, sec-butyl, n-pentyl, 1,1-dimethyl propyl, 1,2-dimethyl propyl, 2,2-dimethyl propyl, 1-ethyl propyl, 2-methyl butyl, 3-methyl butyl, n-hexyl, 1-ethyl-2-methyl propyl, 1,1,2-trimethyl propyl, 1,1-dimethyl butyl, 1,2-dimethyl butyl, 2,2-dimethyl butyl, 1,3-dimethyl butyl, 2-ethyl butyl, 2-methyl pentyl, 3-methyl pentyl, 4-methyl pentyl, 2,3-dimethyl butyl, and the like. The alkyl may be substituted or unsubstituted.

"Alkenyl" refers to the alkyl as defined above consisting of at least two carbon atoms and at least one carbon-carbon double bond, and representative examples comprise, but are not limited to, vinyl, 1-propenyl, 2-propenyl, 1-, 2- or 3-butenyl, 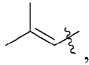 and the like. Preferably, the alkenyl is C₂-C₄ alkenyl. The alkenyl may be optionally substituted or unsubstituted.

"Alkynyl" refers to an aliphatic hydrocarbon group containing one carbon-carbon triple bond, and may be a straight chain or a branched chain. Preferably, the alkynyl is C₂-C₁₀ alkynyl, more preferably, the alkynyl is C₂-C₆ alkynyl, and most preferably, the alkynyl is C₂-C₄ alkynyl. Embodiments of an alkynyl group comprise, but are not limited to, ethynyl, 1-propynyl, 2-propynyl, 1-, 2- or 3-butynyl, and the like. The alkynyl may be substituted or unsubstituted.

"Alkylene" refers to a saturated C₁-C₂₀ linear or branched aliphatic hydrocarbon group, which has 2 residues derived by removing two hydrogen atoms from the same carbon or two different carbon atoms of the parent alkane, and is preferably C₁-C₁₀ alkylene, and more preferably C₁-C₆ alkylene. Embodiments of an alkylene group comprise, but are not limited to, methylene, 1,1-ethylidene, 1,2-ethylidene, 1,1-propylidene, 1,2-propylidene, 1,3-propylidene, 1,4-butylidene, and the like. The alkylene may be substituted or unsubstituted.

"Cycloalkyl" refers to a non-aromatic cyclic alkyl group, wherein one or more ring-forming atoms are carbon atoms, the "cycloalkyl" comprises a monocyclic ring, a polycyclic ring, a fused ring, a bridged ring and a spirocyclic ring, and preferably comprises a 5-7 membered monocyclic ring or a 7-10 membered bicyclic or tricyclic ring. Embodiments of a "cycloalkyl" comprise, but are not limited to, cyclopropyl, cyclopentyl, cyclobutyl, and the like. The cycloalkyl may be substituted or unsubstituted.

"Spiroalkyl" refers to a 5-18 membered polycyclic group with two or more cyclic structures, and single rings sharing one carbon atom (called a spiro atom) with each other, and the ring contains one or more double bonds, but no ring has a fully conjugated π electron aromatic system. Preferably, the spiroalkyl is a 6 to 14 membered group, and more preferably, the spiroalkyl is a 7 to 10 membered group. The spiroalkyl is divided into mono-, di- or multi-spiroalkyl according to a number of shared spiro-atoms between rings, is preferably the mono- and di-spiroalkyl, and preferably is 4 membered/5 membered, 4 membered/6 membered, 5 membered/5 membered or 5 membered/6 membered. Non-limiting embodiments of the "spiroalkyl" comprise, but are not limited to, spiro[4.5]decyl, spiro[4.4]nonyl, spiro[3.5]nonyl, spiro[2.4]heptyl, and the like.

"Fused cycloalkyl" refers to a 5-18 membered full-carbon polycyclic group with two or more cyclic structures sharing one pair of carbon atoms with each other, and one or more rings may contain one or more double bonds, but no ring has a fully conjugated π electron aromatic system. Preferably, the fused cycloalkyl is a 6 to 12 membered group, and more preferably, the fused cycloalkyl is a 7 to 10 membered group. The fused cycloalkyl may be divided into bicyclic, tricyclic, tetracyclic or polycyclic fused cycloalkyl according to a number of constituent rings. The fursed cycloalkyl is preferably the bicyclic or tricyclic fused cycloalkyl, and is more preferably bicycloalkyl with 5 membered/5 membered or 5 memebered/6 membered. Non-limiting embodiments of the "fused cycloalkyl" comprise, but are not limited to, bicyclo[3.1.0]hexyl, bicyclo[3.2.0]heptyl-1-alkenyl, bicyclo[3.2.0]heptyl, decahydronaphthyl, tetrahydrophenyl, and the like.

"Bridged cycloalkyl" refers to a 5-18 membered full-carbon polycyclic group with two or more cyclic structures sharing two carbon atoms not directly attached with each other, and one or more rings may contain one or more double bonds, but no ring has a fully conjugated π electron aromatic system. Preferably, the bridged cycloalkyl is a 6 to 12 memebered group, and more preferably, the bridged cycloalkyl is a 7 to 10 membered group. Preferably, the bridged cycloalkyl is a 6 to 14 membered group, and more preferably, the bridged cycloalkyl is a 7 to 10 membered group. The bridged cycloalkyl may be divided into bicyclic, tricyclic, tetracyclic or polycyclic bridged cycloalkyl according to a number of constituent rings The bridged cycloalkyl is preferably the bicyclic, tricyclic or tetracyclic bridged cycloalkyl, and is more preferably the bicyclic or tricyclic bridged cycloalkyl. Non-limiting embodiments of the "bridged cycloalkyl" comprise, but are not limited to, (1s,4s)-bicyclo [2.2.1] heptyl, bicyclo [3.2.1] octyl, (1s,5s)-bicyclo [3.3.1] nonyl, bicyclo [2.2.2] octyl, (1r,5r)-bicyclo [3.3.2] decyl, and the like.

"Heterocyclyl", ""heterocycloalkyl", "heterocycle" or "heterocyclic" may be used interchangeably in the present application, and each refers to non-aromatic heterocyclyl, wherein one or more ring-forming atoms are selected from heteroatoms: nitrogen, oxygen or S(O) t (wherein t is selected from 0, 1 or 2). The heterocyclyl comprises a monocyclic ring, a polycyclic ring, a fused ring, a bridged ring and a spirocyclic ring. Preferably, the heterocyclyl is a 5-7 membered monocyclic ring or a 7-10 membered bicyclic or tricyclic ring, which may contain 1, 2 or 3 atoms selected from nitrogen, oxygen and/or sulfur. Embodiments of "heterocyclyl" comprise, but are not limited to, morpholinyl, oxetanyl, thiomorpholinyl, tetrahydrofuryl, tetrahydropyranyl, 1,1-dioxo-thiomorpholinyl, piperidinyl, 2-oxo-piperidinyl, pyrrolidinyl, 2-oxo-pyrrolidinyl, piperazine-2-one, 8-oxa-3-aza-bicyclo[3.2.1]octyl, piperazinyl, hexahydropyrimidine, 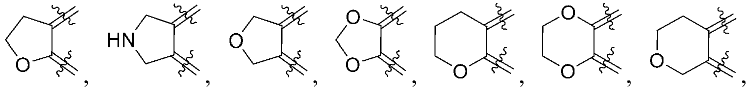 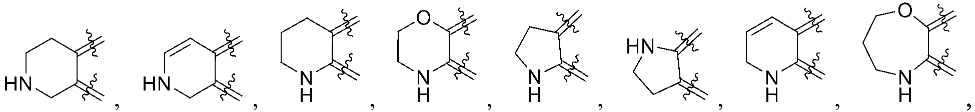 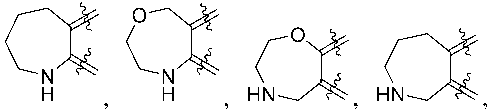 and the like.

The heterocyclyl may be substituted or unsubstituted.

"Spiro-heterocyclyl" refers to a 5-18 membered polycyclic group with two or more cyclic structures, and single rings sharing one atom with each other, and the ring contains one or more double bonds, but no ring has a fully conjugated π electron aromatic system, wherein one or more ring atoms are selected from heteroatoms: nitrogen, oxygen or S(O)t (wherein t is selected from 0, 1 or 2), and the remaining ring atoms are carbon. Preferably, the spiro-heterocyclyl is a 6 to 14 memebered group, and more preferably, the spiro-heterocyclyl is a 7 to 10 memebered group. The spiro-heterocyclyl is divided into mono-, di- or multi-spiro-heterocyclyl according to a number of shared spiro atoms between rings. The spiro-heterocyclyl is preferably the mono- and di-spiro-heterocyclyl, and is more preferably mono-spiro-heterocyclyl with 4 membered/4 membered, 4 membered/5 membered, 3 membered/5 membered, 3 membered/6 membered, 4 membered/6 membered, 5 membered/5 membered or a 5 membered/6 membered. Non-limiting embodiments of the "spiro-heterocyclyl" comprise, but are not limited to, 1,7-dioxane [4.5] decyl, 2-oxa-7-azaspiro [4.4] nonyl, 7-oxaspiro [3.5] nonyl, 5-oxaspiro [2.4] heptyl, 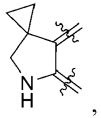 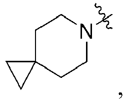 and the like.

"Fused heterocyclyl" refers to a full carbon polycyclic group with two or more cyclic structures sharing one pair of atoms with each other, and one or more rings may contain one or more double bonds, but no ring has a fully conjugated π electron aromatic system, wherein one or more ring atoms are selected from heteroatoms: nitrogen, oxygen or S(O)t (wherein t is selected from 0, 1 or 2), and the remaining ring atoms are carbon. Preferably, the fused heterocyclyl is a 6 to 14 membered group, and more preferably, the fused heterocyclyl is a 7 to 10 membered group. The fused heterocyclyl may be divided into bicyclic, tricyclic, tetracyclic or polycyclic fused heterocyclyl according to a number of constituent rings. The fused heterocyclyl is preferably the bicyclic or tricyclic fused heterocyclyl, and is more preferably the bicyclic fused heterocyclyl with 5 membered/5 membered or 5 membered/6 membered. Non-limiting embodiments of the "fused heterocyclyl" comprise, but are not limited to, octahydropyrrolo [3,4-c] pyrrolyl, octahydro-1H-isoindolyl, 3- azabicyclo [3.1.0] hexyl, octahydrobenzo [b][1,4] dioxine, and the like.

"Bridged heterocyclyl" refers to a 5-18 membered polycyclic group with two or more cyclic structures sharing two atoms not directly attached with each other, and one or more rings may contain one or more double bonds, but no ring has a fully conjugated π electron aromatic system, wherein one or more ring atoms are selected from heteroatoms: nitrogen, oxygen or S(O)ₜ (wherein t is selected from 0, 1 or 2), and the remaining ring atoms are carbon. Preferably, the bridged heterocyclyl is a 6 to 14 membered group, and more preferably, the bridged heterocyclyl is a 7 to 10 membered group. The bridged heterocyclyl may be divided into bicyclic, tricyclic, tetracyclic or polycyclic bridged heterocyclyl according to a number of constituent rings. The bridged heterocyclyl is preferably the bicyclic, tricyclic or tetracyclic bridged heterocyclyl, and is more preferably the bicyclic or tricyclic bridged heterocyclyl. Non-limiting embodiments of the "bridged heterocyclyl" comprise, but are not limited to, 2-azabicyclo[2.2.1]heptyl, 2-azabicyclo[2.2.2]octyl, 2-azabicyclo[3.3.2]decyl, and the like.

"Aryl" refers to a carbocyclic aromatic system containing one or two rings, wherein the rings may be linked together in a fused manner. The term "aryl" comprises monocyclic or bicyclic aryl, such as aromatic groups of phenyl, naphthyl and tetrahydronaphthyl. Preferably, the aryl is C₆-C₁₀ aryl, more preferably, the aryl is phenyl and naphthyl, and most preferably, the aryl is naphthyl. The aryl may be substituted or unsubstituted.

"Heteroaryl" refers to a 5-6 membered aromatic monocyclic ring or a 8-10 membered aromatic bicyclic ring, which may contain 1 to 4 atoms selected from nitrogen, oxygen and/or sulfur. Embodiments of the "heteroaryl" comprise, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothiophenyl, benzimidazolyl, indolyl, isoindolyl, 1,3-dioxo-isoindolyl, quinolyl, indazolyl, benzisothiazolyl, benzoxazolyl, benzisoxazolyl, isothiazolyl, 1H-1,2,4-triazolyl, 4H-1,2,4-triazolyl, pyridyl, pyrimidinyl, pyrazine-2(1H)-keto, pyrimidine-4(3H)-keto, pyridazine-3(2H)-keto, 1H-indolyl, 1H-benzo[d]imidazolyl, 1H-pyrrolo[2,3-c]pyridyl, 3H-imidazo[4,5-c]pyridyl, isoquinolinyl, quinazolinyl, 2H-isoindolyl, furan[3,2-b]pyridyl, furan[2,3-c]pyridyl, thieno[2,3-c]pyridyl, benzofuryl, benzo[b]thienyl, 1H-pyrrolo[3,2-b]pyridyl, 2H-pyrrolo[3,4-c]pyridyl, 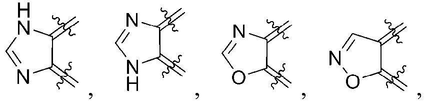 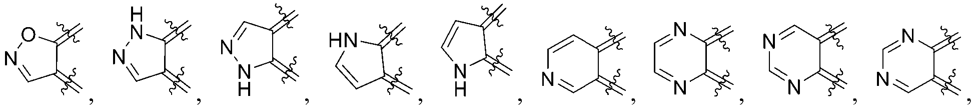 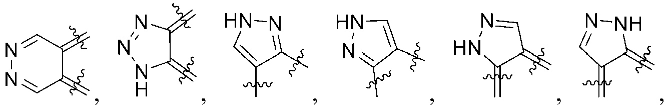 and the like.

The heteroaryl may be substituted or unsubstituted.

"Alkoxy" refers to an "alkyl-O-" group. The alkyl is defined herein. C₁-C₆ alkoxy is prefered. Embodiments of the alkoxy comprise, but are not limited to, methoxy, ethoxy, n-propoxy, isopropoxy, n-butoxy, isobutoxy, tert-butoxy, and the like.

"Nitro" refers to a -NO₂ group.

"Hydroxyl" refers to a -OH group.

"Halogen" refers to fluorine, chlorine, bromine and iodine.

"Amino" refers to -NH₂.

"Cyano" refers to -CN.

"Benzyl" refers to -CH₂-phenyl.

"Carboxyl" refers to -C(O)OH.

"Carboxylate group" refers to -C(O)O-alkyl or -C(O)O-cycloalkyl, wherein the alkyl and the cycloalkyl are defined as above.

"Hydroxyalkyl" refers to hydroxyl-substituted alkyl, wherein the alkyl is defined as above.

"Aminoalkyl" refers to amino-substituted alkyl, wherein the alkyl is defined as above.

"Halogenated alkyl" refers to halogen-substituted alkyl, wherein the alkyl is defined as above.

"Halogenated alkoxy" refers to halogen-substituted alkoxy, wherein the alkoxy is defined as above.

"DMSO" refers to dimethyl sulfoxide.

"BOC" refers to tert-butoxycarbonyl.

"Bn" refers to benzyl.

"THP" refers to 2-tetrahydropyranyl.

"TFA" refers to trifluoroacetic acid

"Ts" refers to p-toluenesulfonyl.

"Leaving group", or leaving group, is an atom or functional group detached from a larger molecule in a chemical reaction, which is a term used in a nucleophilic substitution reaction and an elimination reaction. In the nucleophilic substitution reaction, a reactant attacked by a nucleophilic reagent is called a substrate, and an atom or an atomic group broken together with a pair of electrons from a substrate molecule is called the leaving group. A group that accepts electrons easily and bears negative charges strongly is a good leaving group. When pKa of a conjugated acid of the leaving group is smaller, the leaving group is easier to be separated from other molecules. It is because that, when the pKa of the conjugated acid is smaller, the corresponding leaving group does not need to be combined with other atoms, and the trend of existence in the form of anion (or electrically neutral leaving group) is also enhanced. Common leaving groups comprise, but are not limited to, halogen, methanesulfonyl, -OTs or -OH.

"Substituted" refers to that one or more hydrogen atoms, preferably at most 5 hydrogen atoms, and more preferably 1 to 3 hydrogen atoms, in a group, are independently substituted by a corresponding number of substituents. It goes without saying that the substituents are only in their possible chemical positions, and those skilled in the art can determine (by experiment or theory) possible or impossible substitutions without excessive efforts. For example, amino or hydroxyl with free hydrogen may be unstable when combined with carbon atoms with unsaturated (such as olefinic) bonds.

"Substitution" or "substituted" in the specification, unless otherwise specified, refers to that the group may be substituted by one or more groups selected from alkyl, alkenyl, alkynyl, alkoxy, alkylthio, alkylamino, halogen, sulfhydryl, hydroxyl, nitro, cyano, cycloalkyl, heterocyclyl, aryl, heteroaryl, cycloalkoxy, heterocycloalkoxy, cycloalkylthiol, heterocycloalkylthiol, amino, halogenated alkyl, hydroxyalkyl, carboxyl, carboxylate group, =O, -OR⁶, -C(O)R⁶, -C(O)OR⁶, -NHC(O)R⁶, -NHC(O)OR⁶, -NR⁷R⁸, -C(O)NR⁷R⁸, -CH₂NHC(O)OR⁶, -CH₂NR⁷R⁸ or -S(O)rR⁶;
R⁶ is selected from a hydrogen atom, alkyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁹, -C(O)OR⁹, -OC(O)R⁹, -NR¹⁰R¹¹, -C(O)NR¹⁰R¹¹, -SO₂NR¹⁰R¹¹ or -NR¹⁰C(O)R¹¹;
R⁷ and R⁸ are each independently selected from a hydrogen atom, hydroxyl, halogen, alkyl, alkoxy, cycloalkyl, heterocyclyl, aryl or heteroaryl, wherein the alkyl, the alkoxy, the cycloalkyl, the heterocyclyl, the aryl or the heteroaryl is optionally further substituted by one or more substituents selected from hydroxyl, halogen, nitro, cyano, alkyl, alkoxy, cycloalkyl, heterocyclyl, aryl, heteroaryl, =O, -C(O)R⁹, -C(O)OR⁹, -OC(O)R⁹, -NR¹⁰R¹¹, -C(O)NR¹⁰R¹¹, -SO₂NR¹⁰R¹¹ or -NR¹⁰C(O)R¹¹;
or, R⁷ and R⁸ form a 4-8 membered heterocyclyl together with atoms to which the R⁷ and the R⁸ are attached, wherein the 4-8 membered heterocyclyl contains one or more N, O or S(O)r, and the 4-8 membered heterocyclyl is optionally further substituted by one or more substituents selected from hydroxyl, halogen, nitro, cyano, alkyl, alkoxy, cycloalkyl, heterocyclyl, aryl, heteroaryl, =O, -C(O)R⁹, -C(O)OR⁹, -OC(O)R⁹, -NR¹⁰R¹¹, -C(O)NR¹⁰R¹¹, -SO₂NR¹⁰R¹¹ or -NR¹⁰C(O)R¹¹;
R⁹, R¹⁰ and R¹¹ are each independently selected from a hydrogen atom, alkyl, amino, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or carboxylate group.
r is selected from 0, 1 or 2.

The compound of the present invention may contain an asymmetric center or a chiral center, thus existing in different stereoisomeric forms. It is expected that all stereoisomeric forms of the compound of the present invention comprise, but are not limited to, a diastereomer, an enantiomer, and an atropisomer and a geometric (conformational) isomer and a mixture thereof, such as a racemic mixture, which are all within the scope of the present invention.

Unless otherwise specified, the structure described in the present invention further comprises all isomers of this structure (such as forms of a diastereomer, an enantiomer, and an atropisomer and a geometric (conformational) isomer; such as R and S configurations of asymmetric centers, (Z) and (E) double-bond isomers, and (Z) and (E) conformational isomers). Therefore, a single stereoisomer, and an enantiomeric mixture, a diastereomeric mixture and a geometric (conformational) isomer mixture of the compound of the present invention are all within the scope of the present invention.

"Pharmaceutically acceptable salt" refers to some salts of the above compounds capable of maintaining original biological activity and suitable for medical use. The pharmaceutically acceptable salt of the compound as shown by the general formula (I) may be a metal salt or an amine salt formed with a suitable acid.

"Pharmaceutical composition" refers to a mixture containing one or more compounds described herein or their physiologically acceptable salts or prodrugs and other chemical components, and other components such as physiologically acceptable carriers and excipients. The purpose of the pharmaceutical composition is to promote the administration to organisms, which is beneficial for the absorption of active ingredients, thus exerting biological activity.

### SYNTHESIS METHOD FOR COMPOUND OF THE PRESENT INVENTION

In order to achieve the objective of the present invention, the following technical solution is used in the present invention.

The present invention provides a preparation method for the compound as shown by general formula (I), or the stereoisomer thereof, the tautomer thereof or the pharmaceutically acceptable salt thereof, and the method comprises: 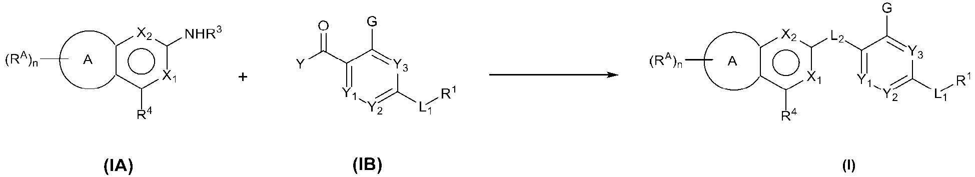
carrying out a condensation reaction on a compound as shown by general formula (IA) and a compound as shown by general formula (IB), and optionally further carrying out a substitution reaction to obtain the compound as shown by general formula (I),
wherein:
   L₂ is selected from 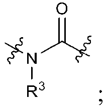
   Y is selected from hydroxyl or chlorine; and
   definitions of ring A, X₁, X₂, Y₁, Y₂, Y₃, L₁, G, R¹, R³, R⁴, R^{A} and n are as described in general formula (I).

### EMBODIMENTS

The present invention is further described in combination with the following embodiments, but these embodiments are not intended to limit the scope of the present invention.

### EXAMPLES

The preparation of a representative compound as shown by formula (I) and the related structural identification data are given in the examples. It must be noted that the following embodiments are used to illustrate the present invention and are not intended to limit the present invention. A ¹H NMR spectrum is measured by a Bruker instrument (400 MHz), and a chemical shift is represented by ppm. Tetramethylsilane is used as an internal standard (0.00 ppm). In a representation method of ¹H NMR: s = single peak, d = double peak, t = triple peak, m = multiple peak, br = broadened, dd = double peak of double peak, and dt = double peak of triple peak. When a coupling constant is provided, the unit of the coupling constant is Hz.

A mass spectrum is measured by an LC/MS instrument, and an ionization mode may be ESI or APCI.

A silica gel plate used for thin-layer chromatography is a Yantai Huanghai HSGF254 or Qingdao GF254 silica gel plate, the silica gel plate used for thin-layer chromatography (TLC) adopts a specification of 0.15 mm~0.2 mm, and a product separated and purified by thin-layer chromatography adopts a specification of 0.4 mm~0.5 mm.

200~300-mesh silica gel of Yantai Huanghai silica gel is generally used as a carrier in column chromatography.

In the following examples, unless otherwise specified, all temperatures are Celsius temperatures. Unless otherwise specified, various starting materials and reagents are commercially available or synthesized according to known methods, and the commercially available raw materials and reagents are directly used without further purification. Unless otherwise specified, manufacturers of the commercially available raw materials and reagents comprise, but are not limited to, Aldrich Chemical Company, ABCR GmbH & Co.KG, Acros Organics, Guangzan Chemical Technology Co., Ltd., Jingyan Chemical Technology Co., Ltd., etc.

CD₃OD: deuterated methanol.

CDCl₃: deuterated chloroform.

DMSO-*d*₆: deuterated dimethyl sulfoxide.

Argon atmosphere means that a reaction bottle is connected with an argon balloon with a volume of about 1 L.

Unless otherwise specified in the embodiments, a solution in the reaction refers to an aqueous solution.

The compound is purified by silica gel column chromatography and reversed-phase column chromatography, wherein an eluent system is selected from an A system: petroleum ether and ethyl acetate system; a B system: dichloromethane and methanol system; a C system: dichloromethane and ethyl acetate system; and a D system: trifluoroacetic acid aqueous solution and acetonitrile system. A volume ratio of a solvent varies according to the polarity of the compound, and may also be adjusted by adding a small amount of acidic or alkaline reagent, such as acetic acid or triethylamine.

### Example 1

### N-(7-(4,4-difluoropiperidin-1-yl)-2,3-dihydrobenzofuran-5-yl)-4-(ethylsulfonamido)-2-(6-azaspi ro[2.5]octan-6-yl)benzamide

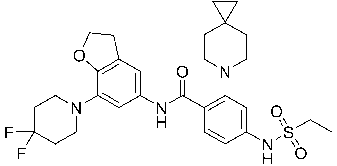

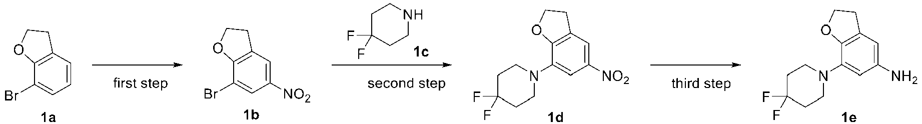

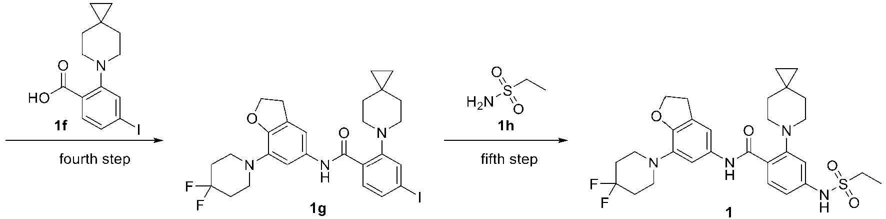

### First step

### 7-Bromo-5-nitro-2,3-dihydrobenzofuran

Nitric acid (1.5 mL) was added dropwise into a solution of 7-bromo-2,3-dihydrobenzofuran **1a** (700 mg, 3.52 mmol, commercially available) in trifluoroacetic acid (3 mL) and stirred at room temperature for 2 hours. LCMS showed that the reaction was completed, and the reaction mixture was added into water (50 mL) dropwise slowly to precipitate a pale yellow solid. After filtered, the filter cake was dried in vacuum to obtain 7-bromo-5-nitro-2,3-dihydrobenzofuran **1b** (750 mg), with a yield of 87.39%.
MS m/z (ESI): 244.0 [M+1].

### Second step

### 4,4-difluoro-1-(5-nitro-2,3-dihydrobenzofuran-7-yl)piperidine

7-bromo-5-nitro-2,3-dihydrobenzofuran **1b** (650 mg, 2.66 mmol) and 4,4-difluorpiperidine **1c** (645.24 mg, 5.33 mmol) were dissolved in toluene (20 mL), then palladium acetate (59.80 mg, 266.35 µmol), R-(+)-1,1'-binaphthyl-2,2'-bis(diphenylphosphine) (497.54 mg, 799.04 µmol) and cesium carbonate (2.60 g, 7.99 mmol) were added, and the mixture was subjected to argon replacement for 3 times, heated to 100°C, and stirred for 16 hours. After the reaction was completed, water (30 mL) was added and extracted with dichloromethane (20 mL×2). The combined organic phase was washed with saturated sodium chloride solution (20 mL), dried over anhydrous sodium sulfate, filtered, and concentrated under reduced pressure. The residue was purified by silica gel column chromatography (eluent: A system) to obtain 4,4-difluoro-1-(5-nitro-2,3-dihydrobenzofuran-7-yl)piperidine **1d** (610 mg), with a yield of 80.57%.
MS m/z (ESI): 285.1 [M+1].

### Third step

### 7-(4,4-difluoropiperidin-1-yl)-2,3-dihydrobenzofuran-5-amine

Ammonium chloride (559.35 mg, 10.55 mmol) and iron powder (589.38 mg, 10.55 mmol) were added into a mixed solution of 4,4-difluoro-1-(5-nitro-2,3-dihydrobenzofuran-7-yl)piperidine **1d** (300 mg, 1.06 mmol) in water (20 mL) and ethanol (20 mL), the mixture was heated to 80°C, and stirred for 2 hours. After the reaction was completed, filtered, and ethyl acetate (30 mL) was added for extraction. The water phase was fuether extracted with ethyl acetate (30 mL×2), and the combined organic phase was washed with saturated sodium chloride solution (30 mL), dried over anhydrous sodium sulfate, and concentrated under reduced pressure to obtain 7-(4,4-difluoropiperidin-1-yl)-2,3-dihydrobenzofuran-5-amine **1e** (220 mg), with a yield of 81.98%.
MS m/z (ESI): 255.1 [M+1].

### Fourth step

### N-(7-(4,4-difluoropiperidin-1-yl)-2,3-dihydrobenzofuran-5-yl)-4-iodo-2-(6-azaspiro[2.5]octan-6-yl)benzamide

7-(4,4-difluoropiperidin-1-yl)-2,3-dihydrobenzofuran-5-amine **1e** (68.34 mg, 268.77 µmol), 2-(6-azaspiro[2.5]octane-6-yl)-4-iodo-benzoic acid **1f** (80 mg, 223.97 µmol, prepared according to the method in the published patent WO2020132648) and 1-methylimidazole (55.84 mg, 671.92 µmol) were added into N,N-dimethylformamide (2 mL), and stirred at room temperature for 30 minutes, then (chloro(dimethylamino)methylene)-dimethylammonium hexafluorophosphate (188.53 mg, 671.92 µmol) was added into the mixture and stirred at room temperature for 2 hours. After the reaction was completed, the reaction mixture was concentrated under reduced pressure. The residue was purified by silica gel column chromatography (eluent: A system) to obtain N-(7-(4,4-difluoropiperidin-1-yl)-2,3-dihydrobenzofuran-5-yl)-4-iodo-2-(6-azaspiro[2.5]octan-6-yl)benzamide **1g** (92 mg), with a yield of 69.22%.
MS m/z (ESI): 594.4 [M+1].

### Fifth step

### N-(7-(4,4-difluoropiperidin-1-yl)-2,3-dihydrobenzofuran-5-yl)-4-(ethylsulfonamido)-2-(6-azaspi ro[2.5]octan-6-yl)benzamide

N-(7-(4,4-difluoropiperidin-1-yl)-2,3-dihydrobenzofuran-5-yl)-4-iodo-2-(6-azaspiro[2.5]oct an-6-yl)benzamide **1g** (80 mg, 134.81 µmol), ethyl sulfonamide **1h** (22.07 mg, 202.21 µmol, commercially available), 2-(methylamino)acetic acid (18.01 mg, 202.21 µmol), cuprous iodide (7.70 mg, 40.44 µmol) and potassium phosphate trihydrate (179.50 mg, 674.03 µmol) were added into N,N-dimethylformamide (2 mL), and the mixture was purged with argon , then heated to 100°C, and stirred for 6 hours. After the reaction was completed, filtered, and the filtrate was purified by a preparative liquid phase (separation column AKZONOBEL Kromasil; 250×21.2 mm I.D.; 5 µm, 20 mL/min; mobile phase A: 0.05%TFA+H₂O, mobile phase B: CH₃CN) to obtain N-(7-(4,4-difluoropiperidin-1-yl)-2,3-dihydrobenzofuran-5-yl)-4-(ethylsulfonamido)-2-(6-azaspi ro[2.5]octan-6-yl)benzamide 1 (31 mg), with a yield of 40.02%.
MS m/z (ESI): 575.3 [M+1].
¹H NMR (400 MHz, DMSO-*d₆*) δ 11.50 (s, 1H), 10.14 (s, 1H), 7.82 (d, J = 8.5 Hz, 1H), 7.42 (d, J = 1.8 Hz, 1H), 7.17 (d, J = 2.1 Hz, 1H), 7.07 - 7.01 (m, 2H), 4.54 (d, J = 8.7 Hz, 2H), 3.19 (qd, J = 8.7, 7.2, 4.1 Hz, 8H), 2.97 (t, J = 5.2 Hz, 4H), 2.09 (tt, J = 13.8, 5.3 Hz, 4H), 1.55 (t, J = 5.1 Hz, 4H), 1.20 (t, J = 7.3 Hz, 3H), 0.35 (s, 4H).

### Example 2

### N-(7-(4,4-difluoropiperidin-1-yl)-2-methyl-1-oxoisoindolin-5-yl)-4-(ethylsulfonamido)-2-(6-aza spiro[2.5]octan-6-yl)benzamide

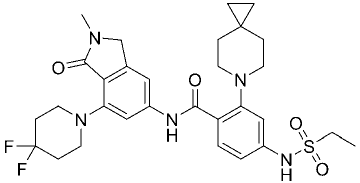

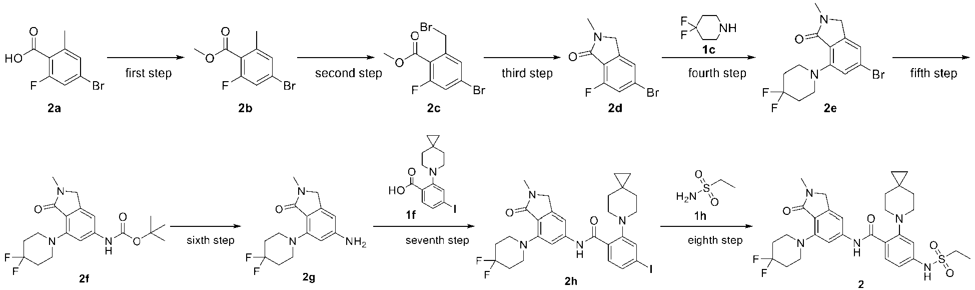

### First step

### Methyl 4-bromo-2-fluoro-6-methylbenzoate

4-Bromo-2-fluoro-6-methylbenzoic acid 2a (1.0 g, 4.29 mmol, commercially available) and potassium carbonate (1.19 g, 8.58 mmol) were dissolved in N,N-dimethylformamide (10 mL), methyl iodide (913.63 mg, 6.44 mmol) was added dropwise at room temperature, and the mixture was stirred for 2 hours. After the reaction was completed, water (20 mL) was added dropwise to the reaction mixture and the obtained mixture was extracted with ethyl acetate (50 mL×2). The combined organic phase was washed with saturated sodium chloride solution (10 mL), dried over anhydrous sodium sulfate, filtered, and concentrated. The residue was purified by silica gel column chromatography (eluent: A system) to obtain methyl 4-bromo-2-fluoro-6-methylbenzoate 2b (1.03 g), with a yield of 97.15%.
MS m/z (ESI): 247.0 [M+1].
¹H NMR (400 MHz, DMSO-*d₆*) δ 7.54 (dd, J = 9.5, 1.8 Hz, 1H), 7.46 (d, J = 1.8 Hz, 1H), 3.87 (s, 3H), 2.33 (s, 3H).

### Second step

### Methyl 4-bromo-2-(bromomethyl)-6-fluorobenzoate

Methyl 4-bromo-2-fluoro-6-methylbenzoate **2b** (500 mg, 2.02 mmol) and azodiisobutyronitrile (33.23 mg, 202.38 µmol) were dissolved in acetonitrile (10 mL), then N-bromosuccinimide (468.26 mg, 2.63 mmol) was added at room temperature. The obtained mixture was heated to 90°C, and stirred for 2 hours. After the reaction was completed, water (20 mL) was added dropwise to the reaction mixture, and the obtained mixture was extracted with ethyl acetate (50 mL×2). The combined organic phase was washed with saturated sodium chloride solution (10 mL), dried over anhydrous sodium sulfate, filtered, and concentrated. The residue was purified by silica gel column chromatography (eluent: A system) to obtain methyl 4-bromo-2-(bromomethyl)-6-fluorobenzoate **2c** (0.53 g), with a yield of 80.34%.
MS m/z (ESI): 326.9 [M+1].

### Third step

### 5-Bromo-7-fluoro-2-methylisoindolin-1-one

Methyl 4-bromo-2-(bromomethyl)-6-fluorobenzoate **2c** (500 mg, 1.53 mmol) was dissolved in methylamine dioxane solution (4 M, 3.83 mL), heated to 40°C, and stirred for 2 hours. After the reaction was completed, water (20 mL) was added dropwise to the reaction mixture, and the obtained system was extracted with ethyl acetate (50 mL×2). The combined organic phase was washed with saturated sodium chloride solution (10 mL), dried over anhydrous sodium sulfate, filtered, and concentrated. The residue was purified by silica gel column chromatography (eluent: A system) to obtain 5-bromo-7-fluoro-2-methylisoindolin-1-one **2d** (130 mg), with a yield of 34.72%.
MS m/z (ESI): 244.0 [M+1].

### Fourth step

### 5-Bromo-7-(4,4-difluoropiperidin-1-yl)-2-methylisoindolin-1-one

5-Bromo-7-fluoro-2-methylisoindolin-1-one **2d** (120 mg, 491.68 µmol) and 4,4-difluoropiperidine **1c** (9.93 mg, 81.95 µmol) were dissolved in dimethyl sulfoxide (5 mL), then N,N-diisopropylethylamine (158.87 mg, 1.23 mmol) was added, heated to 150°C, and stirred for 8 hours. After the reaction was completed, water (20 mL) was added dropwise to the reaction mixture, and the obtinaed mixture was extracted with ethyl acetate (50 mL×2). The combined organic phase was washed with saturated sodium chloride solution (10 mL), dried over anhydrous sodium sulfate, filtered, and concentrated. The residue was purified by silica gel column chromatography (eluent: A system) to obtain 5-bromo-7-(4,4-difluoropiperidin-1-yl)-2-methylisoindolin-1-one **2e** (150 mg), with a yield of 88.38%.
MS m/z (ESI): 344.8 [M+1].

### Fifth step

### Tert-butyl (7-(4,4-difluoropiperidin-1-yl)-2-methyl-1-oxoisoindolin-5-yl)carbamate

5-Bromo-7-(4,4-difluoropiperidin-1-yl)-2-methylisoindolin-1-one **2e** (140 mg, 405.58 µmol) and tert-butyl carbamate (95.02 mg, 811.17 µmol) were dissolved in 1,4-dioxane (10 mL), then palladium acetate (9.11 mg, 40.56 µmol), 4,5-bisdiphenylphosphino-9,9-dimethylxanthene (46.94 mg, 81.12 µmol) and cesium carbonate (396.44 mg, 1.22 mmol) were added, and the mixture was subjected to argon sparging for 5 minutes, heated to 100°C, and stirred for 18 hours. After the reaction was completed, water (20 mL) was added dropwise to the reaction mixture and the obtained mixture was extracted with ethyl acetate (20 mL×2). The combined organic phase was washed with saturated sodium chloride solution (10 mL), dried over anhydrous sodium sulfate, filtered, and concentrated. The residue was purified by silica gel column chromatography (eluent: A system) to obtain tert-butyl (7-(4,4-difluoropiperidin-1-yl)-2-methyl-1-oxoisoindolin-5-yl)carbamate **2f** (150 mg), with a yield of 96.96%.
MS m/z (ESI): 382.0 [M+1].

### Sixth step

### 5-Amino-7-(4,4-difluoropiperidin-1-yl)-2-methylisoindolin-1-one

Tert-butyl (7-(4,4-difluoropiperidin-1-yl)-2-methyl-1-oxoisoindolin-5-yl)carbamate **2f** (150 mg, 393.27 µmol) was dissolved in hydrochloric acid (4 M, 983.18 µL), heated to 60°C, and stirred for 16 hours. After the reaction was completed, water (20 mL) and ethyl acetate (20 mL) was added sequentially to the reaction mixture. A pH value was adjusted to be 8-9 with saturated sodium bicarbonate solution, and the mixture was separated. The organic phase was dried over anhydrous sodium sulfate, filtered, and concentrated to obtain 5-amino-7-(4,4-difluoropiperidin-1-yl)-2-methylisoindolin-1-one **2g** (110 mg), with a yield of 99.43%.
MS m/z (ESI): 281.9 [M+1].

### Seventh step

### N-(7-(4,4-difluoropiperidin-1-yl)-2-methyl-1-oxoisoindolin-5-yl)-4-iodo-2-(6-azaspiro[2.5]octan -6-yl)benzamide

2-(6-azaspiro[2.5]octane-6-yl)-4-iodo-benzoic acid **1f** (130 mg, 363.96 µmol) was dissolved in acetonitrile (10 mL), then 1-methylimidazole (60.50 mg, 727.91 µmol) and tetramethylchloroformamidinium-hexafluorophosphe (204.24 mg, 727.91 µmol) were added, and stirred at room temperature for 1 hour. 5-amino-7-(4,4-difluoropiperidin-1-yl)-2-methylisoindolin-1-one **2g** (112.62 mg, 400.35 µmol) was added, and the obtained mixture was heated to 60°C, and stirred for 4 hours. After the reaction was completed, the reaction mixture was concentrated under reduced pressure. The residue was purified by silica gel column chromatography (eluent: A system) to obtain N-(7-(4,4-difluoropiperidin-1-yl)-2-methyl-1-oxoisoindolin-5-yl)-4-iodo-2-(6-azaspiro[2.5]octan -6-yl)benzamide **2h** (180 mg), with a yield of 79.71%.
MS m/z (ESI): 620.8 [M+1].

### Eighth step

### N-(7-(4,4-difluoropiperidin-1-yl)-2-methyl-1-oxoisoindolin-5-yl)-4-(ethylsulfonamido)-2-(6-aza spiro[2.5]octan-6-yl)benzamide

N-(7-(4,4-difluoropiperidin-1-yl)-2-methyl-1-oxoisoindolin-5-yl)-4-iodo-2-(6-azaspiro[2.5] octan-6-yl)benzamide **2h** (100 mg, 161.17 µmol) and ethyl sulfonamide **1h** (35.18 mg, 322.34 µmol) were dissolved in N,N-dimethylformamide (10 mL), then cuprous iodide (17.89 mg, 80.58 µmol), 2-(methylamino)acetic acid (14.36 mg, 161.17 µmol) and potassium phosphate trihydrate (214.60 mg, 805.84 µmol) were added, and the mixture was subjected to argon sparging for 5 minutes, heated to 100°C, and stirred for 18 hours. After the reaction was completed, water (20 mL) was added dropwise to the reaction mixture and the obtained system was extracted with ethyl acetate (20 mL×2). The combined organic phase was washed with saturated sodium chloride solution (10 mL), dried over anhydrous sodium sulfate, filtered, and concentrated. The residue was purified by a preparative liquid phase (separation column AKZONOBEL Kromasil; 250×21.2 mm I.D.; 5 µm, 20 mL/min; mobile phase A: 0.05%TFA+H₂O, mobile phase B: CH₃CN) to obtain N-(7-(4,4-difluoropiperidin-1-yl)-2-methyl-1-oxoisoindolin-5-yl)-4-(ethylsulfonamido)-2-(6-aza spiro[2.5]octan-6-yl)benzamide **2** (45.3 mg), with a yield of 45.92%.
MS m/z (ESI): 602.3 [M+1].
¹H NMR (400 MHz, DMSO-*d₆*) δ 11.87 (s, 1H), 10.18 (s, 1H), 7.82 (d, J = 8.5 Hz, 1H), 7.74 (s, 1H), 7.17 (d, J = 2.1 Hz, 1H), 7.11 (s, 1H), 7.05 (dd, J = 8.5, 2.1 Hz, 1H), 4.37 (s, 2H), 3.20 (q, J = 7.3 Hz, 2H),3.30(m, 4H), 3.00 (s, 3H), 2.97 (t, J = 5.3 Hz, 4H), 2.15 (ddt, J = 25.7, 18.1, 8.9 Hz, 4H), 1.55 (t, J = 5.3 Hz, 4H), 1.21 (t, J = 7.3 Hz, 3H), 0.34 (s, 4H).

### Example 3

### 4-(Ethylsulfonamido)-N-(7-(piperidin-1-yl)-2,3-dihydrobenzofuran-5-yl)-2-(6-azaspiro[2.5]octa n-6-yl)benzamide

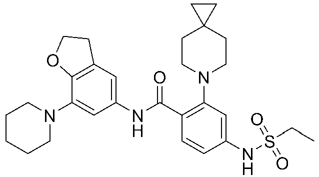

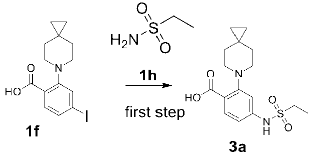

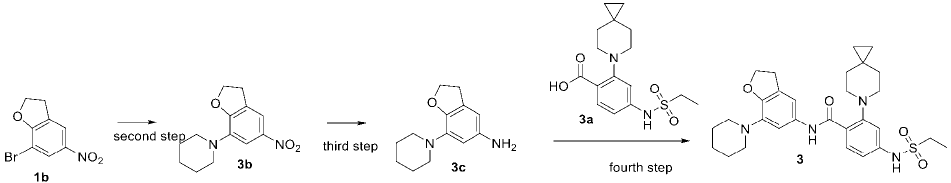

### First step

### 4-(Ethylsulfonamido)-2-(6-azaspiro[2.5]octan-6-yl)benzoic acid

2-(6-Azaspiro[2.5]octane-6-yl)-4-iodo-benzoic acid **1f** (1.0 g, 2.80 mmol) and ethyl sulfonamide **1h** (458.36 mg, 4.20 mmol) were dissolved in N,N-dimethylformamide (10 mL), then cuprous iodide (266.60 mg, 1.40 mmol), sarcosine (249.43 mg, 2.80 mmol) and potassium phosphate (3.73 g, 14.00 mmol) were added, and the mixture was purged with argon, heated to 110°C, and stirred for 6 hours. After cooling to room temperature, the reaction mixture was poured into ice water (200 mL), and a pH value was adjusted to 6 with dilute hydrochloric acid (2 M). The mixture was extracted with dichloromethane (200 mL×3). The combined organic phase was washed with saturated sodium chloride solution (50 mL), dried over sodium sulfate, filtered, and concentrated under reduced pressure. The residue was purified by silica gel column chromatography (eluent: A system) to obtain 4-(ethylsulfonamido)-2-(6-azaspiro[2.5]octan-6-yl)benzoic acid 3a (0.75 g), with a yield of 79.16%.
MS m/z (ESI): 339.4 [M+1].

### Second step

### 1-(5-Nitro-2,3-dihydrobenzofuran-7-yl)piperidine

At room temperature, 7-bromo-5-nitro-2,3-dihydrobenzofuran **1b** (488 mg, 2.00 mmol) and piperidine (340.53 mg, 4.00 mmol) were dissolved in toluene (20 mL), then palladium acetate (44.89 mg, 199.97 µmol), R-(+)-1,1'-binaphthyl-2,2'-bis(diphenylphosphine) (373.54 mg, 599.90 µmol) and cesium carbonate (1.95 g, 6.00 mmol) were added, and the mixture was purged with argon for 3 times, heated to 100°C, and stirred for 16 hours. Water (20 mL) was added and extracted with ethyl acetate (20 mL×3). The combined organic phase was washed with saturated sodium chloride solution (20 mL), dried over anhydrous sodium sulfate, and concentrated under reduced pressure. The residue was purified by silica gel column chromatography (eluent: A system) to obtain 1-(5-nitro-2,3-dihydrobenzofuran-7-yl)piperidine **3b** (310 mg), with a yield of 62%.
MS m/z (ESI): 249.1 [M+1].

### Third step

### 7-(Piperidin-1-yl)-2,3-dihydrobenzofuran-5-amine

At room temperature, palladium on carbon (100 mg, 805.55 µmol, 10%) was added into a solution of 1-(5-nitro-2,3-dihydrobenzofuran-7-yl)piperidine **3b** (0.2 g, 805.55 µmol) in methanol (20 mL), and the mixture was purged with hydrogen, then heated to 50°C, and stirred for 4 hours. The reaction mixture was filtered, and the filtrate was concentrated under reduced pressure to obtain 7-(piperidin-1-yl)-2,3-dihydrobenzofuran-5-amine **3c** (152 mg), with a yield of 86%. The crude product was directly used in the next reaction.
MS m/z (ESI): 219.0 [M+1].

### Fourth step

### 4-(Ethylsulfonamido)-N-(7-(piperidin-1-yl)-2,3-dihydrobenzofuran-5-yl)-2-(6-azaspiro[2.5]octa n-6-yl)benzamide

At room temperature, tetramethylchloroformamidinium-hexafluorophosphe (345.98 mg, 664.85 µmol), 4-(ethylsulfonamido)-2-(6-azaspiro[2.5]octan-6-yl)benzoic acid **3a** (150 mg, 443.23 µmol) and N,N-diisopropylethylamine (171.85 mg, 1.33 mmol) were added into N,N-dimethylformamide (3 mL), and stirred at room temperature for 10 minutes, then 7-(piperidin-1-yl)-2,3-dihydrobenzofuran-5-amine **3c** (116.11 mg, 531.88 µmol) was added into the mixture, heated to 65°C, and stirred for 2 hours. Water (20 mL) was added and extracted with ethyl acetate (10 mL×3). The combined organic phase was washed with saturated sodium chloride solution (10 mL), dried over anhydrous sodium sulfate, and concentrated under reduced pressure. The residue was separated by a preparative liquid phase (separation column AKZONOBEL Kromasil; 250×21.2 mm I.D.; 5 µm, 20 mL/min; mobile phase A: 0.05%TFA+H₂O, mobile phase B: CH₃CN) to obtain 4-(ethylsulfonamido)-N-(7-(piperidin-1-yl)-2,3-dihydrobenzofuran-5-yl)-2-(6-azaspiro[2.5]octan -6-yl)benzamide 3 (38 mg), with a yield of 16%.
MS m/z (ESI): 539.3 [M+1].
¹H NMR (400 MHz, DMSO-*d₆*) δ 11.62 (s, 1H), 10.11 (s, 1H), 7.84 (d, J = 8.5 Hz, 1H), 7.40 (s, 1H), 7.17 (d, J = 2.1 Hz, 1H), 7.04 (dd, J = 8.4, 2.1 Hz, 1H), 6.94 (d, J = 2.0 Hz, 1H), 4.50 (t, J = 8.7 Hz, 2H), 3.23 - 3.11 (m, 4H), 3.10-3.0 (m, 4H), 3.0-2.90 (m, 4H), 1.67-1.59 (m, 4H), 1.59-1.46 (m, 6H), 1.20 (t, J = 7.3 Hz, 3H), 0.40-0.28 (m, 4H).

Examples 4-7 were synthesized according to the synthesis methods in Examples 1-3 of the present invention. Structures and characterization data of Examples 4-7 are shown in the following table:

| **Serial number and structure of examples** | **MS m/z (ESI)** | **¹H NMR** |
|---|---|---|
| 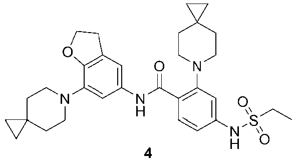 | 565.3 [M+1] | ¹H NMR (400 MHz, DMSO-*d₆*) δ 11.55 (s, 1H), 10.13 (s, 1H), 7.82 (d, J = 8.5 Hz, 1H), 7.44 (s, 1H), 7.40 (s, 1H), 7.18 (d, J = 2.2 Hz, 1H), 7.05 (dd, J = 8.5, 2.1 Hz, 1H), 4.60 (d, J = 8.6 Hz, 2H), 3.30-3.25 (m, 4H), 3.25-3.10 (m, 4H), 3.05-2.89 (m, 4H), 1.66-1.4 (m, 8H), 1.21 (t, J = 7.3 Hz, 3H), 0.40-0.37 (m, 4H), 0.37-0.30 (m, 4H). |
| 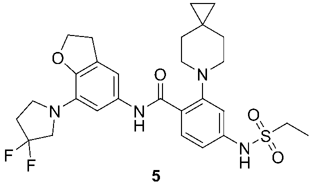 | 561.3 [M+1] | ¹H NMR (400 MHz, DMSO-*d₆*) δ 11.52 (s, 1H), 10.11 (s, 1H), 7.82 (d, J = 8.5 Hz, 1H), 7.30 (d, J = 1.7 Hz, 1H), 7.17 (d, J = 2.1 Hz, 1H), 7.03 (dd, J = 8.5, 2.0 Hz, 1H), 6.82 (d, J = 2.0 Hz, 1H), 4.50 (t, J = 8.7 Hz, 2H), 3.70 (t, J = 13.5 Hz, 2H), 3.47 (t, J = 7.1 Hz, 2H), 3.24 - 3.11 (m, 4H), 3.05-2.90 (m, 4H), 2.44 (dt, J = 14.6, 7.1 Hz, 2H), 1.60-1.50 (m, 4H), 1.21 (t, J = 7.3 Hz, 3H), 0.40-0.28 (m, 4H). |
| 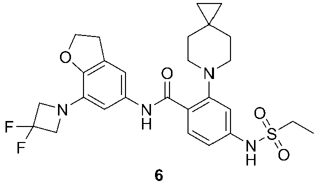 | 547.6 [M+1] | ¹H NMR (400 MHz, DMSO-*d₆*) δ 11.39 (s, 1H), 10.12 (s, 1H), 7.80 (d, J = 8.5 Hz, 1H), 7.17 (dd, J = 5.4, 2.0 Hz, 2H), 7.02 (dd, J = 8.5, 2.1 Hz, 1H), 6.79 (d, J = 2.0 Hz, 1H), 4.50 (t, J = 8.7 Hz, 2H), 4.25 (t, J = 12.3 Hz, 4H), 3.22 (m, 5H), 2.96 (t, J = 5.3 Hz, 4H), 1.53 (s, 3H), 1.20 (t, J = 7.3 Hz, 3H), 0.36 (s, 4H). |
| 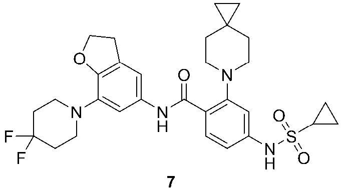 | 587.3 [M+1] | ¹H NMR (400 MHz, DMSO-*d₆*) δ 11.59 (s, 1H), 10.09 (s, 1H), 7.83 (d, J = 8.5 Hz, 1H), 7.43 (d, J = 1.9 Hz, 1H), 7.20 (d, J = 2.2 Hz, 1H), 7.09 (m, 2H), 4.53 (t, J = 8.7 Hz, 2H), 3.25 (m, 6H), 2.97 (t, J = 5.3 Hz, 4H), 2.80 (m, 1H), 2.15 (m, 4H), 1.56 (s, 4H), 0.99 (d, J = 4.6 Hz, 4H), 0.36 (s, 4H). |

### Example 8

### N-(7-cyclopropyl-2,3-dihydrobenzofuran-5-yl)-4-(ethylsulfonamido)-2-(6-azaspiro[2.5]octan-6-yl)benzamide

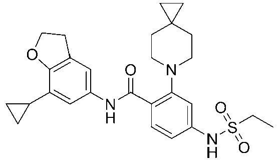

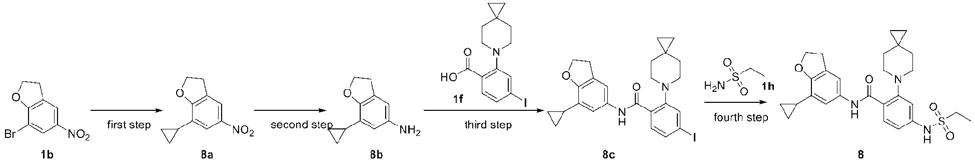

### First step

### 7-Cyclopropyl-5-nitro-2,3-dihydrobenzofuran

At room temperature, 7-bromo-5-nitro-2,3-dihydrobenzofuran **1b** (0.5 g, 2.05 mmol), cyclopropylboric acid (351.98 mg, 4.10 mmol), potassium phosphate trihydrate (1.64 g, 6.15 mmol) and tetrakis(triphenylphosphine)palladium (236.75 mg, 204.88 µmol) were added into a mixed solvent of ethanol (2 mL), water (2 mL) and toluene (10 mL), and the mixture was purged with argon for 3 times, heated to 100°C, and stirred for 16 hours. Water (20 mL) was added and extracted with ethyl acetate (20 mL×3). The combined organic phase was washed with saturated sodium chloride solution (20 mL), dried over anhydrous sodium sulfate, and concentrated under reduced pressure. The residue was purified by silica gel column chromatography (eluent: A system) to obtain 7-cyclopropyl-5-nitro-2,3-dihydrobenzofuran **8a** (255 mg), with a yield of 61%.
MS m/z (ESI): 205.9 [M+1].

### Second step

### 7-Cyclopropyl-2,3-dihydrobenzofuran-5-amine

At room temperature, palladium on carbon (20 mg, 1.24 mmol, 10%) was added into a solution of 7-cyclopropyl-5-nitro-2,3-dihydrobenzofuran **8a** (255 mg, 1.24 mmol) in methanol (5 mL), and the mixture was purged with hydrogen and stirred at room temperature for 2 hours. The reaction mixture was filtered, and the filtrate was concentrated under reduced pressure to obtain 7-cyclopropyl-2,3-dihydrobenzofuran-5-amine **8b** (195 mg), with a yield of 90%. The crude product was directly used in the next reaction.
MS m/z (ESI): 176.1 [M+1].

### Third step

### N-(7-cyclopropyl-2,3-dihydrobenzofuran-5-yl)-4-iodo-2-(6-azaspiro[2.5]octan-6-yl)benzamide

A solution of 2-(6-azaspiro[2.5]octane-6-yl)-4-iodo-benzoic acid **1f** (0.1 g, 279.97 µmol) and N,N-dimethylformamide (0.1 mL) in dichloromethane (3 mL) was cooled by ice bath, then oxalyl chloride (71.07 mg, 559.93 µmol) was added dropwise slowly, and the obtained mixture was stirred at room temperature for 2 hours. The mixture was concentrated under reduced pressure. The residue was added into a solution of 7-cyclopropyl-2,3-dihydrobenzofuran-5-amine **8b** (53.96 mg, 307.96 µmol) and triethylamine (84.99 mg, 839.90 µmol) in dichloromethane (3 mL), and the obtained mixture was stirred at room temperature for 1 hour. Water (10 mL) was added into and extracted with ethyl acetate (10 mL×3). The combined organic phase was washed with saturated sodium chloride solution (10 mL), dried over anhydrous sodium sulfate, and concentrated under reduced pressure. The residue was purified by silica gel column chromatography (eluent: A system) to obtain N-(7-cyclopropyl-2,3-dihydrobenzofuran-5-yl)-4-iodo-2-(6-azaspiro[2.5]octan-6-yl)benzamide **8c** (121 mg), with a yield of 84%.
MS m/z (ESI): 514.8 [M+1].

### Fourth step

### N-(7-cyclopropyl-2,3-dihydrobenzofuran-5-yl)-4-(ethylsulfonamido)-2-(6-azaspiro[2.5]octan-6-yl)benzamide

At room temperature, N-(7-cyclopropyl-2,3-dihydrobenzofuran-5-yl)-4-iodo-2-(6-azaspiro[2.5]octan-6-yl)benzamide **8c** (150 mg, 291.60 µmol), ethyl sulfonamide **1h** (47.74 mg, 437.41 µmol), 2-(methylamino)acetic acid (38.97 mg, 437.41 µmol), cuprous iodide (16.66 mg, 87.48 µmol) and potassium phosphate trihydrate (232.97 mg, 874.81 µmol) were added into N,N-dimethylformamide (2 mL), and the mixture was purged with argon, then heated to 100°C, and stirred for 6 hours. Water (20 mL) was added and extracted with ethyl acetate (10 mL×3). The combined organic phase was washed with saturated sodium chloride solution (10 mL), dried over anhydrous sodium sulfate, and concentrated under reduced pressure. The residue was purified by a preparative liquid phase (separation column AKZONOBEL Kromasil; 250×21.2 mm I.D.; 5 µm, 20 mL/min; mobile phase A: 0.05%NH₄CO₃+H₂O, mobile phase B: CH₃CN) to obtain N-(7-cyclopropyl-2,3-dihydrobenzofuran-5-yl)-4-(ethylsulfonamido)-2-(6-azaspiro[2.5]octan-6-yl)benzamide **8** (18 mg), with a yield of 12%.
MS m/z (ESI): 495.9 [M+1].
¹H NMR (400 MHz, DMSO-*d₆*) δ 11.39 (s, 1H), 10.09 (s, 1H), 7.80 (d, J = 8.5 Hz, 1H), 7.57 (d, J = 2.0 Hz, 1H), 7.15 (d, J = 2.1 Hz, 1H), 7.02 (dd, J = 8.5, 2.1 Hz, 1H), 6.89 (d, J = 2.1 Hz, 1H), 4.52 (t, J = 8.6 Hz, 2H), 3.23 - 3.09 (m, 4H), 3.0-2.90 (m, 4H), 1.91 (tt, J = 8.4, 5.2 Hz, 1H), 1.55-1.48 (m, 4H), 1.20 (t, J = 7.3 Hz, 3H), 0.97 - 0.87 (m, 2H), 0.69 - 0.61 (m, 2H), 0.37-0.31 (m, 4H).

Example 9 was synthesized according to the synthesis method in Example 8 of the present invention. The structure and characterization data of Example 9 are shown in the following table:

| **Serial number andstructure of example** | **MS m/z (ESI)** | **¹H NMR** |
|---|---|---|
| 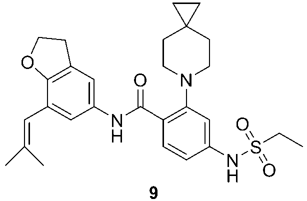 | 510.2 [M+1] | ¹H NMR (400 MHz, DMSO-*d₆*) δ 11.41 (s, 1H), 10.13 (s, 1H), 7.87 - 7.75 (m, 2H), 7.18 (dd, J = 6.2, 2.1 Hz, 2H), 7.04 (dd, J = 8.5, 2.2 Hz, 1H), 6.14 (s, 1H), 4.51 (t, J = 8.7 Hz, 2H), 3.24-3.13 (m, 4H), 3.0-2.94 (m, 4H), 1.88 (s, 3H), 1.85 (s, 3H), 1.57-1.5 (m, 4H), 1.21 (t, J = 7.3 Hz, 3H), 0.37-0.3 (m, 4H). |

### Example 10

### 4-(Ethylsulfonamido)-N-(7-isobutyl-2,3-dihydrobenzofuran-5-yl)-2-(6-azaspiro[2.5]octan-6-yl)b enzamide

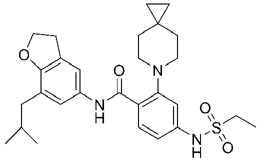

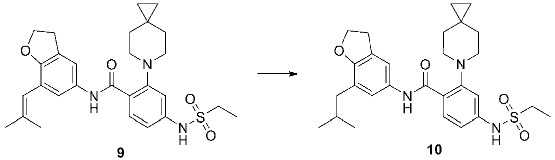

At room temperature, 4-(ethylsulfonamido)-N-(7-(2-methylprop-1-en-1-yl)-2,3-dihydrobenzofuran-5-yl)-2-(6-azaspiro [2.5]octan-6-yl)benzamide 9 (50 mg, 98.10 µmol) and 10% palladium on carbon hydrogenation catalyst (10 mg, 98.10 µmol) were added into methanol (3 mL), and the mixture was purged with hydrogen three times, then stirred at room temperature for 4 hours. The reaction mixture was filtered, the filter cake was washed with methanol (10 mL), and the filtrate was concentrated under a reduce pressure. The cresidue was purified by a preparative liquid phase (separation column AKZONOBEL Kromasil; 250×21.2 mm I.D.; 5 µm, 20 mL/min; mobile phase A: 0.05%TFA+H₂O, mobile phase B: CH₃CN) to obtain 4-(ethylsulfonamido)-N-(7-isobutyl-2,3-dihydrobenzofuran-5-yl)-2-(6-azaspiro[2.5]octan-6-yl)b enzamide 10 (2.5 mg), with a yield of 5%.
MS m/z (ESI): 512.3 [M+1].
¹H NMR (400 MHz, DMSO-*d₆*) δ 11.36 (s, 1H), 10.12 (s, 1H), 7.83 (d, J = 8.5 Hz, 1H), 7.68 (d, J = 2.0 Hz, 1H), 7.18 (d, J = 2.1 Hz, 1H), 7.12 - 7.00 (m, 2H), 4.49 (t, J = 8.6 Hz, 2H), 3.24-3.1 (m, 4H), 3.03-2.94 (m, 4H), 2.37 (d, J = 7.1 Hz, 2H), 1.95-1.80 (m, 1H), 1.57-1.49 (m, 4H), 1.21 (t, J = 7.3 Hz, 3H), 0.89 (d, J = 6.6 Hz, 6H), 0.37-0.31 (m, 4H).

### Example 11

### N-(7-(4,4-difluoro-1-hydroxycyclohexyl)-2,3-dihydrobenzofuran-5-yl)-4-(ethylsulfonamido)-2-( 6-azaspiro[2.5]octan-6-yl)benzamide

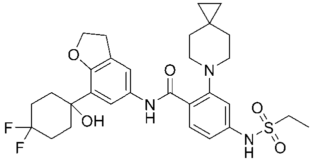

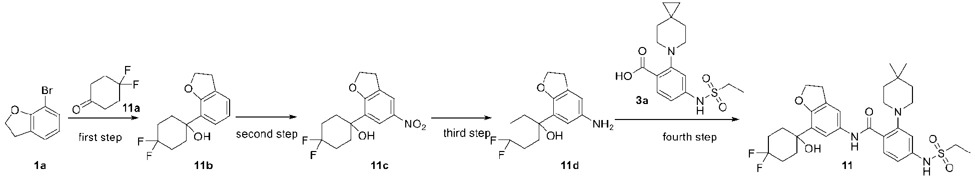

### First step

### 1-(2,3-dihydrobenzofuran-7-yl)-4,4-difluorocyclohexan-1-ol

At -78°C, n-butyl lithium (965.44 mg, 15.07 mmol, 2.5 M) was added dropwise into a solution of 7-bromo-2,3-dihydrobenzofuran **1a** (2 g, 10.05 mmol) in tetrahydrofuran (50 mL) and stirred at -78°C for 0.5 hour, then 4,4-difluorocyclohexanone **11a** (1.48 g, 11.05 mmol) was added dropwise into the reaction mixture. The obtained mixture was stirred at -78°C for 0.5 hour, then warmed to room temperature slowly, and stirred for 2 hours. The mixture was quenched with saturated ammonium chloride aqueous solution (20 mL), ethyl acetate (30 mL×2) was added for extraction. The combined organic phase was washed with saturated sodium chloride aqueous solution, dried over anhydrous sodium sulfate, and concentrated under reduced pressure. The residue was purified by silica gel chromatography (eluent: A system) to obtain 1-(2,3-dihydrobenzofuran-7-yl)-4,4-difluorocyclohexan-1-ol **11b** (1.6 g), with a yield of 63%.
MS m/z (ESI): 237.1 [M-17].
¹H NMR (400 MHz, DMSO-*d₆*) δ 7.34 (dd, J = 7.8, 1.4 Hz, 1H), 7.11 (dd, J = 7.3, 1.3 Hz, 1H), 6.82 (t, J = 7.5 Hz, 1H), 5.11 (s, 1H), 4.50 (t, J = 8.7 Hz, 2H), 3.13 (t, J = 8.7 Hz, 2H), 2.45-2.30 (m, 2H), 2.30 - 2.04 (m, 2H), 1.96 - 1.80 (m, 2H), 1.60-1.50 (m, 2H).

### Second step

### 4,4-Difluoro-1-(5-nitro-2,3-dihydrobenzofuran-7-yl)cyclohexan-1-ol

At room temperature, nitric acid (2 mL) was added into a solution of 1-(2,3-dihydrobenzofuran-7-yl)-4,4-difluorocyclohexan-1-ol **11b** (500 mg, 1.97 mmol) in acetic acid (5 mL) and stirred for 4 hours. Water (20 mL) was added dropwise slowly and the obtained mixture was extracted with ethyl acetate (20 mL×3). The combined organic phase was washed with saturated sodium chloride solution, dried over anhydrous sodium sulfate, and concentrated under reduced pressuret. The residue was purified by silica gel column chromatography (eluent: A system) to obtain 4,4-difluoro-1-(5-nitro-2,3-dihydrobenzofuran-7-yl)cyclohexan-1-ol **11c** (274 mg), with a yield of 46%.
MS m/z (ESI): 300.1 [M+1].
¹H NMR (400 MHz, DMSO-*d₆*) δ 8.34 (d, J = 2.6 Hz, 1H), 8.16 - 7.88 (m, 1H), 4.75 (t, J = 8.9 Hz, 2H), 3.28 (t, J = 8.8 Hz, 2H), 2.40 (td, J = 13.7, 4.2 Hz, 2H), 2.29 - 2.07 (m, 2H), 2.00 - 1.85 (m, 2H), 1.63 - 1.49 (m, 2H).

### Third step

### 1-(5-Amino-2,3-dihydrobenzofuran-7-yl)-4,4-difluorocyclohexan-1-ol

At room temperature, a palladium on carbon hydrogenation catalyst (20 mg, 183.78 µmol, 10%) was added into a solution of 4,4-difluoro-1-(5-nitro-2,3-dihydrobenzofuran-7-yl)cyclohexan-1-ol **11c** (55 mg, 183.78 µmol) in methanol (5 mL), and the mixture was purged with hydrogen and stirred at room temperature for 16 hours. The reaction mixture was filtered, and the filtrate was concentrated under reduced pressure to obtain 1-(5-amino-2,3-dihydrobenzofuran-7-yl)-4,4-difluorocyclohexan-1-ol **11d** (41 mg), with a yield of 83%.
MS m/z (ESI): 270.1 [M+1].

### Fourth step

### N-(7-(4,4-difluoro-1-hydroxycyclohexyl)-2,3-dihydrobenzofuran-5-yl)-4-(ethylsulfonamido)-2-( 6-azaspiro[2.5]octan-6-yl)benzamide

At room temperature, tetramethylchloroformamidinium-hexafluorophosphe (92.26 mg, 177.29 µmol), 4-(ethylsulfonamido)-2-(6-azaspiro[2.5]octan-6-yl)benzoic acid **3a** (40 mg, 118.20 µmol) and N,N-diisopropylethylamine (45.83 mg, 354.59 µmol) were added into N,N-dimethylformamide (3 mL), and stirred at room temperature for 10 minutes, then 1-(5-amino-2,3-dihydrobenzofuran-7-yl)-4,4-difluorocyclohexan-1-ol **11d** (35.01 mg, 130.02 µmol) was added into the mixture, and the obtained mixture was heated to 65°C, stirred for 2 hours. Water (10 mL) was added and the obtained mixture was extracted with ethyl acetate (10 mL×3). The combined organic phase was washed with saturated sodium chloride solution (10 mL), dried over anhydrous sodium sulfate, and concentrated under reduced pressure. The residue was separated by a preparative liquid phase (separation column AKZONOBEL Kromasil; 250×21.2 mm I.D.; 5 µm, 20 mL/min; mobile phase A: 0.05%TFA+H₂O, mobile phase B: CH₃CN) to obtain N-(7-(4,4-difluoro-1-hydroxycyclohexyl)-2,3-dihydrobenzofuran-5-yl)-4-(ethylsulfonamido)-2-( 6-azaspiro[2.5]octan-6-yl)benzamide **11** (31 mg), with a yield of 41%.
MS m/z (ESI): 590.3 [M+1].
¹H NMR (400 MHz, DMSO-*d₆*) δ 11.37 (s, 1H), 10.19 (s, 1H), 7.89 (d, J = 8.5 Hz, 1H), 7.70 (s, 1H), 7.56 (s, 1H), 7.25 (s, 1H), 7.09 (d, J = 8.5, 2.0 Hz, 1H), 4.53 (t, J = 8.7 Hz, 2H), 3.30-3.13 (m, 4H), 3.13-3.0 (m, 4H), 2.39 (td, J = 13.7, 4.2 Hz, 2H), 2.31 - 2.07 (m, 2H), 1.98-1.83 (m, 2H), 1.67 - 1.52 (m, 6H), 1.21 (t, J = 7.3 Hz, 3H), 0.37-0.31 (m, 4H).

Examples 12-21 were synthesized according to the synthesis methods in Examples 1 and 11 of the present invention. Structures and characterization data of Examples 12-21 are shown in the following table:

| **Serial number and structure of example** | **MS m/z (ESI)** | **¹H NMR** |
|---|---|---|
| 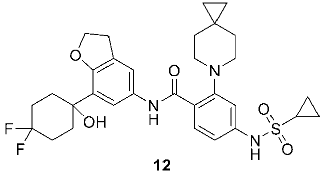 | 602.3 [M+1] | ¹H NMR (400 MHz, DMSO-*d₆*) δ 11.44 (s, 1H), 10.18 (s, 1H), 7.92 (d, J = 8.5 Hz, 1H), 7.71 (s, 1H), 7.58 (s, 1H), 7.31 (s, 1H), 7.13 (d, J = 8.4 Hz, 1H), 4.53 (t, J = 8.7 Hz, 2H), 3.17 (t, J = 8.7 Hz, 2H), 3.15-3.0 (m, 4H), 2.85-2.70 (m, 1H), 2.45-2.32 (m, 2H), 2.30-2.10 (m, 2H), 2.0-1.8 (m, 2H), 1.70-1.50 (m, 6H), 1.1-0.9 (m, 4H), 0.37-0.31 (m, 4H). |
| 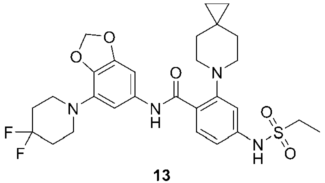 | 577.3 [M+1] | ¹H NMR (400 MHz, DMSO-*d₆*) δ 11.58 (s, 1H), 7.80 (d, J = 8.5 Hz, 1H), 7.16 (dd, J = 6.9, 1.9 Hz, 2H), 7.03 (dd, J = 8.6, 2.0 Hz, 1H), 6.85 (d, J = 1.9 Hz, 1H), 5.97 (s, 2H), 3.20-3.15 (m, 4H), 3.0-2.88 (m, 5H), 2.18-2.0 (m, 5H), 1.60-1.47 (m, 4H), 1.21 (t, J = 7.3 Hz, 3H), 0.37-0.31 (m, 4H). |
| 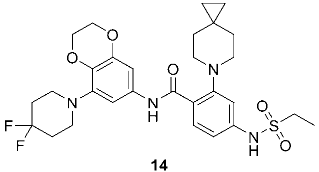 | 591.3 [M+1] | ¹H NMR (400 MHz, DMSO-*d₆*) δ 11.55 (s, 1H), 10.15 (s, 1H), 7.83 (d, J = 8.5 Hz, 1H), 7.18 (d, J = 2.2 Hz, 1H), 7.11 (d, J = 2.3 Hz, 1H), 7.05 (dd, J = 8.5, 2.1 Hz, 1H), 6.92 (d, J = 2.5 Hz, 1H), 4.3-4.2 (m, 4H), 3.19 (q, J = 7.3 Hz, 2H), 3.15-3.08 (m, 4H), 3.0-2.92 (m, 4H), 2.2-2.0 (m, 4H), 1.68-1.47 (m, 4H), 1.21 (t, J = 7.3 Hz, 3H), 0.37-0.31 (m, 4H). |
| 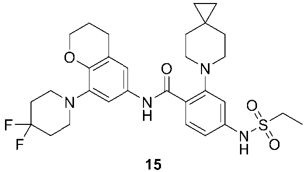 | 589.3 [M+1] | ¹H NMR (400 MHz, DMSO-*d₆*) δ 11.49 (s, 1H), 10.14 (s, 1H), 7.83 (d, J = 8.5 Hz, 1H), 7.29 (d, J = 2.3 Hz, 1H), 7.18 (d, J = 2.1 Hz, 1H), 7.13 - 7.01 (m, 2H), 4.20 - 4.12 (m, 2H), 3.19 (q, J = 7.3 Hz, 2H), 3.15-3.05 (m, 4H), 3.0-2.93 (m, 4H), 2.74 (t, J = 6.5 Hz, 2H), 2.18-2.0 (m, 4H), 1.98-1.87 (m, 2H), 1.59 - 1.52 (m, 4H), 1.20 (t, J = 7.3 Hz, 3H), 0.37-0.31 (m, 4H). |
| 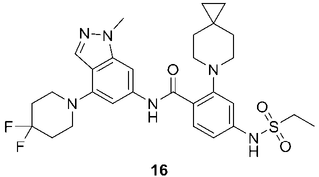 | 587.3 [M+1] | ¹H NMR (400 MHz, DMSO-*d₆*) δ 11.97 (s, 1H), 10.13 (s, 1H), 8.05 (s, 1H), 7.97 (s, 1H), 7.88 (d, J = 8.5 Hz, 1H), 7.19 (d, J = 2.1 Hz, 1H), 7.07 (dd, J = 8.5, 2.1 Hz, 1H), 6.57 - 6.53 (m, 1H), 3.95 (s, 3H), 3.43 (t, J = 5.8 Hz, 4H), 3.21 (q, J = 7.3 Hz, 2H), 2.98 (t, J = 5.3 Hz, 4H), 2.27-2.12 (m, 4H), 1.59 (s, 4H), 1.21 (t, J = 7.3 Hz, 3H), 0.35 (s, 4H). |
| 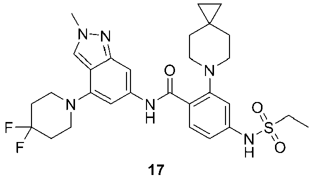 | 586.9 [M+1] | NA |
| 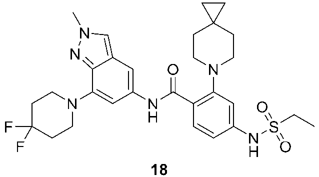 | 587.3 [M+1] | ¹H NMR (400 MHz, DMSO-*d₆*) δ 11.74 (s, 1H), 10.13 (s, 1H), 8.23 (s, 1H), 7.95 (d, J = 1.5 Hz, 1H), 7.87 (d, J = 8.5 Hz, 1H), 7.19 (d, J = 2.1 Hz, 1H), 7.06 (dd, J = 8.5, 2.1 Hz, 1H), 6.63 (d, J = 1.7 Hz, 1H), 4.13 (s, 3H), 3.73 - 3.65 (m, 4H), 3.20 (q, J = 7.3 Hz, 2H), 2.98 (t, J = 5.2 Hz, 4H), 2.21-2.10 (m, 4H), 1.59 (t, J = 5.1 Hz, 4H), 1.21 (t, J = 7.4 Hz, 3H), 0.36 (s, 4H). |
| 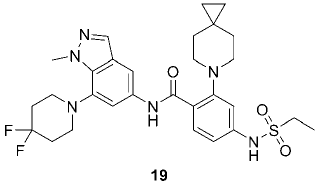 | 586.9 [M+1] | ¹H NMR (400 MHz, DMSO-*d₆*) δ 11.72 (s, 1H), 10.15 (s, 1H), 8.10 (d, J = 1.6 Hz, 1H), 8.01 (s, 1H), 7.85 (d, J = 8.5 Hz, 1H), 7.29 (d, J = 1.7 Hz, 1H), 7.19 (d, J = 2.1 Hz, 1H), 7.06 (dd, J = 8.5, 2.1 Hz, 1H), 5.09 (s, 4H), 4.28 (s, 3H), 3.19 (t, J = 7.3 Hz, 2H), 2.99 (t, J = 5.3 Hz, 4H), 2.34-2.12 (m, 4H), 1.65-1.47 (m, 4H), 1.21 (t, J = 7.3 Hz, 3H), 0.35 (s, 4H). |
| 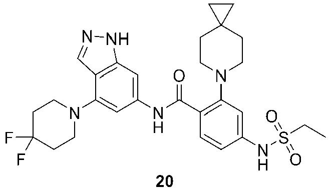 | 572.9 [M+1] | ¹H NMR (400 MHz, DMSO-*d₆*) δ 12.92 (s, 1H), 11.93 (s, 1H), 10.16 (s, 1H), 8.07 (s, 1H), 7.94 (s, 1H), 7.87 (d, J = 8.4 Hz, 1H), 7.19 (d, J = 2.1 Hz, 1H), 7.06 (dd, J = 8.4, 2.0 Hz, 1H), 6.59 (s, 1H), 3.42 (t, J = 5.5 Hz, 4H), 3.20 (q, J = 7.3 Hz, 2H), 2.98 (t, J = 5.2 Hz, 4H), 2.27 - 2.13 (m, 4H), 1.59 (s, 4H), 1.21 (t, J = 7.3 Hz, 3H), 0.35 (s, 4H). |
| 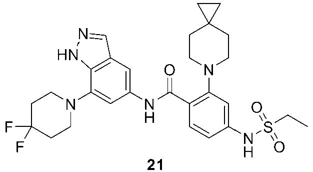 | 572.9 [M+1] | NA |

### Example 22

### N-(7-(4,4-difluoropiperidin-1-yl)-2-methylbenzo[d]oxazol-5-yl)-4-(ethylsulfonamido)-2-(6-azas piro[2.5]octan-6-yl)benzamide

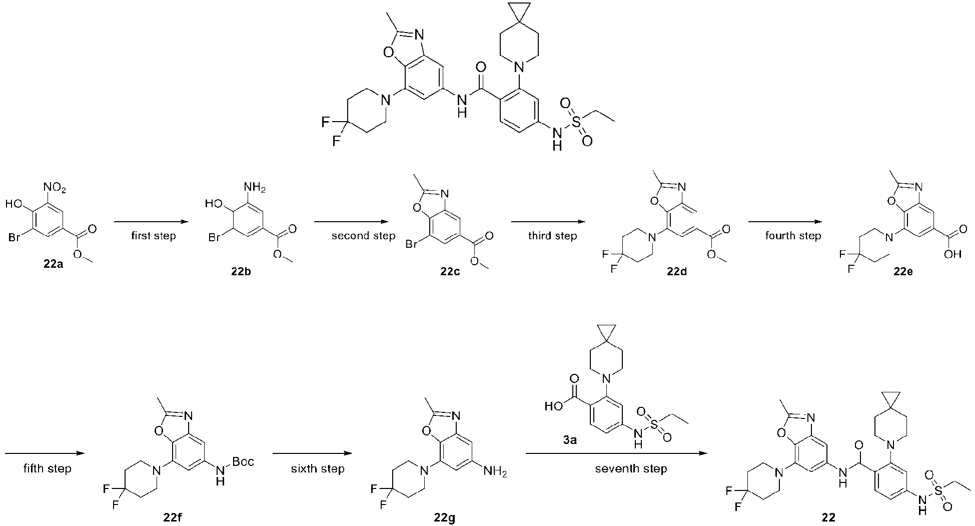

### First step

### Methyl 3-amino-5-bromo-4-hydroxybenzoate

At room temperature, methyl 3-bromo-4-hydroxy-5-nitrobenzoate 22a (1.0 g, 3.62 mmol, commercially available) was dissolved in N,N-dimethylformamide (5 mL), and stannous chloride (2.40 g, 12.68 mmol) was added, and the mixture waspurged with argon 3 times, and stirred at room temperature for 3 hours, and mass spectrum showed that the reaction was completed. Water (30 mL) was added and extracted with ethyl acetate (50 mL×3). The combined organic phase was washed with saturated sodium chloride solution, dried over anhydrous sodium sulfate, filtered, and concentrated. The residue was purified by silica gel column chromatography (eluent: A system) to obtain methyl 3-amino-5-bromo-4-hydroxybenzoate 22b (0.85 g), with a yield of 95.36%.
MS m/z (ESI): 246.0 [M+1].

### Second step

### Methyl 7-bromo-2-methylbenzo[d]oxazole-5-carboxylate

At room temperature, methyl 3-amino-5-bromo-4-hydroxybenzoate 22b (850 mg, 3.45 mmol) was dissolved in 1,1,1-triethoxyethane (8.85 g, 54.55 mmol, 10 mL), and 4-methylbenzenesulfonic acid (59.49 mg, 345.45 µmol) was added, and the mixture was purged with argon 3 times, heated to 80°C, and stirred for 1 hour, and mass spectrum showed that the reaction was completed. The reaction solution was concentrated to dryness. The residue was purified by silica gel column chromatography (eluent: A system) to obtain methyl 7-bromo-2-methylbenzo[d]oxazole-5-carboxylate **22c** (0.75 g), with a yield of 80.39%.
MS m/z (ESI): 270.0 [M+1].

### Third step

### Methyl 7-(4,4-difluoropiperidin-1-yl)-2-methylbenzo[d]oxazole-5-carboxylate

At room temperature, methyl 7-bromo-2-methylbenzo[d]oxazole-5-carboxylate **22c** (750 mg, 2.78 mmol) and 4,4-difluoropiperidine (504.55 mg, 4.17 mmol) were dissolved in toluene (20 mL), and tri(dibenzylideneacetone)palladium (127.15 mg, 138.85 µmol), R-(+)-1,1'-binaphthyl-2,2'-bis(diphenylphosphine) (172.91 mg, 277.70 µmol) and cesium carbonate (2.71 g, 8.33 mmol) were added, and the mixture was purged with argon, heated to 100°C, and stirred for 18 hours, and mass spectrum showed that the reaction was completed. Water (20 mL) was added, and the obtained mixture was stirred for layering and extracted with ethyl acetate (30 mL×2). The combined organic phase was washed with saturated sodium chloride solution, dried over anhydrous sodium sulfate, filtered, and concentrated under reduced pressure. The residue was purified by silica gel column chromatography (eluent: A system) to obtain methyl 7-(4,4-difluoropiperidin-1-yl)-2-methylbenzo[d]oxazole-5-carboxylate **22d** (0.65 g), with a yield of 75.43%.
MS m/z (ESI): 311.1 [M+1].

### Fourth step

### 7-(4,4-Difluoropiperidin-1-yl)-2-methylbenzo[d]oxazole-5-carboxylic acid

At room temperature, methyl 7-(4,4-difluoropiperidin-1-yl)-2-methylbenzo[d]oxazole-5-carboxylate **22d** (600 mg, 1.93 mmol) was dissolved in methanol (5 mL) and tetrahydrofuran (5 mL), lithium hydroxide solution (2 M, 4.83 mL) was added, and the obtained mixture was stirred at room temperature for 4 hours, and mass spectrum showed that the reaction was completed. The reaction mixture was concentrated and then water (20 mL) was added, a pH value was adjusted to be 3-4 with a hydrochloric acid solution (1 M), and the obtained mixture was extracted with ethyl acetate (30 mL×3). The combined organic phase was washed with saturated sodium chloride solution, dried over anhydrous sodium sulfate, filtered, and concentrated under reduced pressure to obtain 7-(4,4-difluoropiperidin-1-yl)-2-methylbenzo[d]oxazole-5-carboxylic acid 22e (0.53 g), with a yield of 92.52%.
MS m/z (ESI): 297.1 [M+1].

### Fifth step

### Tert-butyl (7-(4,4-difluoropiperidin-1-yl)-2-methylbenzo[d]oxazol-5-yl)carbamate

At room temperature, 7-(4,4-difluoropiperidin-1-yl)-2-methylbenzo[d]oxazole-5-carboxylic acid 22e (200 mg, 675.06 µmol) was dissolved in tetrahydrofuran (10 mL), triethylamine (102.46 mg, 1.01 mmol) and diphenylphosphoryl azide (278.67 mg, 1.01 mmol) were added, and stirred at room temperature for 3 hours, and mass spectrum showed that the reaction was completed. Tert-butanol (50 mL) was added to the reaction mixture, the obtained mixure was heated to 80°C, and stirred for 20 hours. The reaction mixture was concentrated under reduced pressure to dryness. The residue was purified by silica gel column chromatography (eluent: A system) to obtain tert-butyl (7-(4,4-difluoropiperidin-1-yl)-2-methylbenzo[d]oxazol-5-yl)carbamate 22f (100 mg), with a yield of 40.32%.
MS m/z (ESI): 368.1 [M+1].

### Sixth step

### 7-(4,4-Difluoropiperidin-1-yl)-2-methylbenzo[d]oxazol-5-amine

At room temperature, tert-butyl (7-(4,4-difluoropiperidin-1-yl)-2-methylbenzo[d]oxazol-5-yl)carbamate **22f** (90 mg, 244.97 µmol) was dissolved in dichloromethane (5 mL), trifluoroacetic acid (55.86 mg, 489.94 µmol, 5 mL) was added dropwise, and the obtained mixture was stirred at room temperature for 1 hour, and mass spectrum showed that the reaction was completed. The reaction mixture was concentrated under reduced pressure to dryness and water (10 mL) was added, a pH value was adjusted to 7-8 with saturated sodium bicarbonate solution, and the mixture was extracted with ethyl acetate (30 mL×3). The combined organic phase was washed with saturated sodium chloride solution, dried over anhydrous sodium sulfate, filtered, and concentrated to obtain 7-(4,4-difluoropiperidin-1-yl)-2-methylbenzo[d]oxazol-5-amine **22g** (50 mg), with a yield of 76.37%.
MS m/z (ESI): 268.1 [M+1].

### Seventh step

### N-(7-(4,4-difluoropiperidin-1-yl)-2-methylbenzo[d]oxazol-5-yl)-4-(ethylsulfonamido)-2-(6-azas piro[2.5]octan-6-yl)benzamide

At room temperature, 4-(ethylsulfonamido)-2-(6-azaspiro[2.5]octan-6-yl)benzoic acid **3a** (50 mg, 147.74 µmol) was dissolved in acetonitrile (5 mL), 1-methylimidazole (36.39 mg, 443.23 µmol) and N,N,N',N'-tetramethylchloroformamidinium hexafluorophosphate (124.36 mg, 443.23 µmol) were added, and stirred at room temperature for 1 hour. 7-(4,4-difluoropiperidin-1-yl)-2-methylbenzo[d]oxazol-5-amine **22g** (51.33 mg, 192.07 µmol) was added, the mixture was heated to 60°C, and stirred for 1 hour, and mass spectrum showed that the reaction was completed. The reaction mixure was concentrated under reduced pressure to dryness, ethyl acetate (30 mL) was added, the obtained mixture was washed with saturated sodium bicarbonate solution (10 mL×2) and saturated sodium chloride solution, dried over anhydrous sodium sulfate, filtered, and concentrated under reduced pressure. The residue was purified by a preparative liquid phase (separation column AKZONOBEL Kromasil; 250×21.2 mm I.D.; 5 µm, 20 mL/min; mobile phase A: 0.05% NH₄CO₃+H₂O, mobile phase B: CH₃CN), and freeze-dried to obtain N-(7-(4,4-difluoropiperidin-1-yl)-2-methylbenzo[d]oxazol-5-yl)-4-(ethylsulfonamido)-2-(6-azas piro[2.5]octan-6-yl)benzamide **22** (5.7 mg), with a yield of 5.2%.
MS m/z (ESI): 588.3 [M+1].
¹H NMR (400 MHz, DMSO-*d₆*) δ 11.70 (s, 1H), 10.15 (s, 1H), 7.84 (d, J = 8.5 Hz, 1H), 7.75 (d, J = 1.7 Hz, 1H), 7.18 (s, 2H), 7.04 (dd, J = 8.5, 2.1 Hz, 1H), 3.49 (t, J = 5.7 Hz, 4H), 3.20 (q, J = 7.3 Hz, 2H), 3.04-2.92 (m, 4H), 2.60 (s, 3H), 2.22 - 2.10 (m, 4H), 1.66-1.43 (m, 4H), 1.21 (t, J = 7.3 Hz, 3H), 0.35 (s, 4H).

### Example 23

### N-(7-(4,4-difluoropiperidin-1-yl)-1-oxoisoindolin-5-yl)-4-(ethylsulfonamido)-2-(6-azaspiro[2.5] octan-6-yl)benzamide

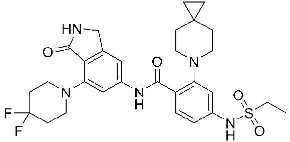

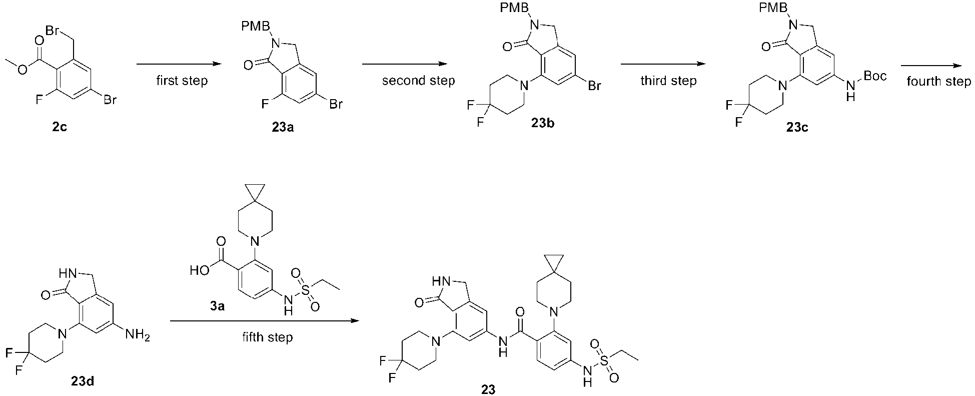

### First step

### 5-Bromo-7-fluoro-2-(4-methoxybenzyl)isoindolin-1-one

At room temperature, methyl 4-bromo-2-(bromomethyl)-6-fluorobenzoate 2c (1.0 g, 3.07 mmol) and (4-methoxyphenyl)methylamine (631.27 mg, 4.60 mmol) were dissolved in N,N-dimethylformamide (10 mL), N,N-diisopropylethylamine (793.00 mg, 6.14 mmol) was added, the obtained mixture was heated to 40°C, and stirred for 10 hours, and mass spectrum showed that the reaction was completed. Water (30 mL) was added to the reaction mixture, and the obtained mixture was extracted with dichloromethane (50 mL×2). The combined organic phase was washed with saturated sodium chloride solution, dried over anhydrous sodium sulfate, filtered, and concentrated under reduced pressure. The residue was purified by silica gel column chromatography (eluent: A system) to obtain 5-bromo-7-fluoro-2-(4-methoxybenzyl)isoindolin-1-one **23a** (0.79 g), with a yield of 73.53%.
MS m/z (ESI): 349.8 [M+1].

### Second step

### 5-Bromo-7-(4,4-difluoropiperidin-1-yl)-2-(4-methoxybenzyl)isoindolin-1-one

At room temperature, 5-bromo-7-fluoro-2-(4-methoxybenzyl)isoindolin-1-one **23a** (450 mg, 1.29 mmol) and 4,4-difluoropiperidine (233.48 mg, 1.93 mmol) were dissolved in dimethyl sulfoxide (10 mL), N,N-diisopropylethylamine (332.17 mg, 2.57 mmol) was added, the obtained mixture was heated to 150°C, and stirred for 6 hours, and mass spectrum showed that the reaction was completed. Water (20mL) was added to the reaction mixture and the obtained mixture was extracted with ethyl acetate (30 mL×2). The combined organic phase was washed with saturated sodium chloride solution, dried over anhydrous sodium sulfate, filtered, and concentrated. The residue was purified by silica gel column chromatography (eluent: A system) to obtain 5-bromo-7-(4,4-difluoropiperidin-1-yl)-2-(4-methoxybenzyl)isoindolin-1-one **23b** (0.44 g), with a yield of 75.8%.
MS m/z (ESI): 450.8 [M+1].

### Third step

### Tert-butyl (7-(4,4-difluoropiperidin-1-yl)-2-(4-methoxybenzyl)-1-oxoisoindolin-5-yl)carbamate

At room temperature, the 5-bromo-7-(4,4-difluoropiperidin-1-yl)-2-(4-methoxybenzyl)isoindolin-1-one **23b** (400 mg, 886.32 µmol) and tert-butyl carbamate (207.66 mg, 1.77 mmol) were dissolved in 1,4-dioxane (10 mL), and palladium acetate (19.90 mg, 88.63 µmol), 4,5-bisdiphenylphosphino-9,9-dimethylxanthene (102.57 mg, 177.26 µmol) and cesium carbonate (866.34 mg, 2.66 mmol) were added, and the mixture was purged with argon, heated to 100°C, and stirred for 18 hours, and mass spectrum showed that the reaction was completed. Water (20mL) was added to the reaction mixture and the obtained mixture was extracted with ethyl acetate (30 mL×2). The combined organic phase was washed with saturated sodium chloride solution, dried over anhydrous sodium sulfate, filtered, and concentrated under reduced pressure. The residue was purified by silica gel column chromatography (eluent: A system) to obtain tert-butyl (7-(4,4-difluoropiperidin-1-yl)-2-(4-methoxybenzyl)-1-oxoisoindolin-5-yl)carbamate **23c** (0.4 g), with a yield of 92.57%.
MS m/z (ESI): 488.0 [M+1].

### Fourth step

### 5-amino-7-(4,4-difluoropiperidin-1-yl)isoindolin-1-one

At room temperature, tert-butyl (7-(4,4-difluoropiperidin-1-yl)-2-(4-methoxybenzyl)-1-oxoisoindolin-5-yl)carbamate **23c** (400 mg, 820.45 µmol) was dissolved in trifluoroacetic acid (4 mL), and stirred at room temperature for 18 hours, methanesulfonic acid (2 mL) was added, the mixture was heated to 60°C, and stirred for 3 hours, and mass spectrum showed that the reaction was completed. After cooling to room temperature, the reaction mixture was concentrated to dryness. Water (20mL) was added to the residue, a pH value was adjusted to 8-9 with saturated sodium carbonate solution, and the mixture was extracted with ethyl acetate (30 mL×3). The combined organic phase was washed with saturated sodium chloride solution, dried over anhydrous sodium sulfate, filtered, and concentrated under reduced pressure to obtain 5-amino-7-(4,4-difluoropiperidin-1-yl)isoindolin-1-one **23d** (0.2 g), with a yield of 91.21%.
MS m/z (ESI): 268.1 [M+1].

### Fifth step

### N-(7-(4,4-difluoropiperidin-1-yl)-1-oxoisoindolin-5-yl)-4-(ethylsulfonamido)-2-(6-azaspiro[2.5] octan-6-yl)benzamide

At room temperature, 4-(ethylsulfonamido)-2-(6-azaspiro[2.5]octan-6-yl)benzoic acid **3a** (150 mg, 443.23 µmol) was dissolved in acetonitrile (10 mL), 1-methylimidazole (110.51 mg, 1.33 mmol) and N,N,N',N'-tetramethylchloroformamidinium hexafluorophosphate (373.09 mg, 1.33 mmol) were added, and the obtained mixture was stirred at room temperature for 1 hour, 5-amino-7-(4,4-difluoropiperidin-1-yl)isoindolin-1-one **23d** (130.31 mg, 487.56 µmol) was added, the mixture was heated to 60°C, and stirred for 2 hours, and mass spectrum showed that the reaction was completed. The reaction solution was purified by a preparative liquid phase (separation column AKZONOBEL Kromasil; 250×21.2 mm I.D.; 5 µm, 20 mL/min; mobile phase A: 0.05% NH₄CO₃+H₂O, mobile phase B: CH₃CN) to obtain N-(7-(4,4-difluoropiperidin-1-yl)-1-oxoisoindolin-5-yl)-4-(ethylsulfonamido)-2-(6-azaspiro[2.5] octan-6-yl)benzamide **23** (43.0 mg), with a yield of 15.75%.
MS m/z (ESI): 588.3 [M+1].
¹H NMR (400 MHz, DMSO-*d₆*) δ 11.88 (s, 1H), 9.82 (s, 1H), 8.18 (s, 1H), 7.82 (d, J = 8.4 Hz, 1H), 7.74 (s, 1H), 7.17 (d, J = 2.1 Hz, 1H), 7.08 (s, 1H), 7.05 (dd, J = 8.5, 2.1 Hz, 1H), 4.28 (s, 2H), 3.44-3.22 (m, 4H), 3.20 (q, J = 7.3 Hz, 2H), 2.97 (t, J = 5.2 Hz, 4H), 2.23-2.09 (m, 4H), 1.61 - 1.50 (m, 4H), 1.21 (t, J = 7.3 Hz, 3H), 0.35 (s, 4H).

Examples 24-26 were synthesized according to the synthesis methods in Examples 1 and 11 of the present invention. Structures and characterization data of Examples 24-26 are shown in the following table:

| **Serial number andstructure of example** | **MS m/z (ESI)** | **¹H NMR** |
|---|---|---|
| 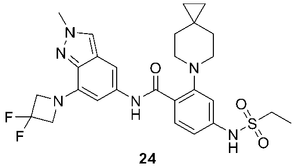 | 558.8 [M+1] | ¹H NMR (400 MHz, DMSO-*d₆*) δ 11.61 (s, 1H), 10.13 (s, 1H), 8.20 (s, 1H), 7.85 (d, J = 8.5 Hz, 1H), 7.72 (d, J = 1.6 Hz, 1H), 7.18 (d, J = 2.2 Hz, 1H), 7.05 (dd, J = 8.5, 2.1 Hz, 1H), 6.35 (d, J = 1.7 Hz, 1H), 4.50 (t, J = 12.2 Hz, 4H), 4.11 (s, 3H), 3.20 (s, 2H), 2.98 (t, J = 5.2 Hz, 4H), 1.65-1.49 (m, 4H), 1.21 (t, J = 7.3 Hz, 3H), 0.36 (s, 4H). |
| 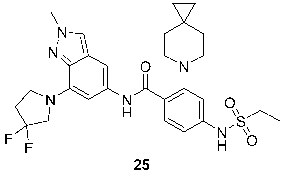 | 572.9 [M+1] | ¹H NMR (400 MHz, DMSO-*d₆*) δ 11.74 (s, 1H), 10.15 (s, 1H), 8.19 (s, 1H), 7.89 (d, J = 8.6 Hz, 1H), 7.77 (s, 1H), 7.21 (d, J = 2.1 Hz, 1H), 7.07 (dd, J = 8.6, 2.1 Hz, 1H), 6.26 (s, 1H), 4.19-4.13 (m, 2H), 4.12 (s, 3H), 3.80 (t, J = 7.2 Hz, 2H), 3.20 (q, J = 7.3 Hz, 2H), 2.99 (t, J = 5.3 Hz, 4H), 2.61 - 2.55 (m, 2H), 1.62 (d, J = 6.0 Hz, 4H), 1.21 (t, J = 7.3 Hz, 3H), 0.37 (s, 4H). |
| 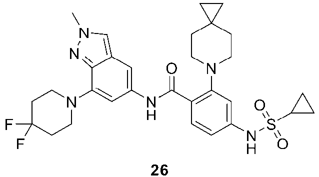 | 598.9 [M+1] | ¹H NMR (400 MHz, DMSO-*d₆*) δ 11.83 (s, 1H), 10.09 (s, 1H), 8.23 (s, 1H), 7.96 (d, J = 1.5 Hz, 1H), 7.89 (d, J = 8.5 Hz, 1H), 7.23 (d, J = 2.1 Hz, 1H), 7.09 (dd, J = 8.5, 2.1 Hz, 1H), 6.63 (d, J = 1.7 Hz, 1H), 4.13 (s, 3H), 3.69 (t, J = 5.7 Hz, 4H), 2.98 (t, J = 5.2 Hz, 4H), 2.78-2.71 (m, 1H), 2.22-2.11 (m, 4H), 1.66-1.53 (m, 4H), 1.03 - 0.97 (m, 4H), 0.36 (s, 4H). |

Examples 27-28 were synthesized according to the synthesis method in Example 2 of the present invention. Structures and characterization data of Examples 27-28 are shown in the following table:

| **Serial number and structure of example** | **MS m/z (ESI)** | **¹H NMR** |
|---|---|---|
| 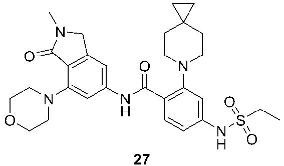 | 567.9 [M+1] | ¹H NMR (400 MHz, DMSO-*d₆*) δ 12.08 (s, 1H), 10.03 (s, 1H), 7.82 (d, J = 8.5 Hz, 1H), 7.72 (s, 1H), 7.14 (s, 1H), 7.06 (s, 1H), 7.01 (d, J = 8.6 Hz, 1H), 4.37 (s, 2H), 3.82 - 3.74 (m, 4H), 3.25-3.19 (m, 4H), 3.17-3.12 (m, 2H), 2.99 (s, 3H), 2.98-2.92 (m, 4H), 1.57 (s, 4H), 1.20 (t, J = 7.3 Hz, 3H), 0.36 (s, 4H). |
| 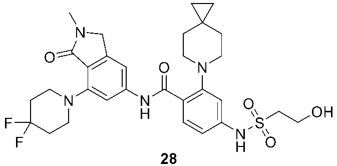 | 617.9 [M+1] | ¹H NMR (400 MHz, DMSO-*d₆*) δ 11.91 (s, 1H), 10.11 (s, 1H), 7.82 (d, J = 8.5 Hz, 1H), 7.74 (s, 1H), 7.21 - 7.09 (m, 2H), 7.03 (dd, J = 8.6, 2.0 Hz, 1H), 4.95 (s, 1H), 4.37 (s, 2H), 3.76 (t, J = 6.5 Hz, 2H), 3.38-3.32 (m, 4H), 3.32-3.31 (m, 2H), 3.00 (s, 3H), 2.97 (t, J = 5.2 Hz, 4H), 2.23 - 2.10 (m, 4H), 1.55 (t, J = 5.4 Hz, 4H), 0.35 (s, 4H). |

Example 29 was synthesized according to the synthesis method in Example 1 of the present invention. The structure and characterization data of Example 29 are shown in the following table:

| **Serial number and structure of example** | **MS m/z (ESI)** | **¹H NMR** |
|---|---|---|
| 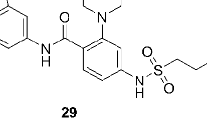 | 591.3 [M+1] | ¹H NMR (400 MHz, DMSO-*d₆*) δ 11.53 (s, 1H), 10.06 (s, 1H), 7.82 (d, J = 8.5 Hz, 1H), 7.42 (s, 1H), 7.15 (d, J = 2.1 Hz, 1H), 7.12 - 6.96 (m, 2H), 4.95 (t, J = 5.6 Hz, 1H), 4.53 (t, J = 8.7 Hz, 2H), 3.75 (q, J = 6.3 Hz, 2H), 3.3 - 3.2 (m, 2H), 3.27-3.12 (m, 6H), 3.0-2.9 (m, 4H), 2.15-2.0 (m, 4H), 1.65-1.46 (m, 4H), 0.37-0.31 (m, 4H). |

### Biological evaluation

### Test Example 1. Determination of inhibition of the compounds of the present invention on OVCAR-3 cell proliferation

Influences of the compounds of the present invention on OVCAR-3 cell proliferation were determined by the following method. OVCAR-3 cells (containing TP53 R248Q mutation) were purchased from the Cell Bank of Shanghai Institute of Biochemistry and Cell Biology, Chinese Academy of Sciences, and cultured in an RPMI 1640 culture medium containing 10% fetal bovine serum, 100 U penicillin and 100 µg/mL streptomycin. A cell viability was determined by a CellTiter-Glo^{®} Luminescent Cell Viability Assay kit (Promega, article number G7573).

An experimental method was operated according to steps in the instruction of the kit, which was briefly described as follows: a test compound was dissolved in DMSO first to prepare a 10 mM stock solution, then the stock solution was diluted with the above RPMI 1640 culture medium to prepare a test sample, and a final concentration of the compound was in a range of 1000 nM-0.015 nM. Cells in a logarithmic growth phase were inoculated into a 96-well cell culture plate at a density of 1000 cells per well, cultured in a 5% CO₂ incubator at 37°C overnight, and then the test compound was added to continuously culture for 72 hours. After the culture was ended, 50 µL of CellTiter-Glo detection solution was added to each well, the plates were shaken for 5 minutes, and then allowed to stand for 10 minutes, and subsequently, a luminous value of each well of sample was read by a microplate reader in a Luminescence mode. By comparing with a numerical value of a control group (0.3% DMSO), a percentage inhibition rate of the compound at each concentration was calculated, and then nonlinear regression analysis was carried out based on concentration logarithm-inhibition rate of the compound in GraphPad Prism 5 software, so as to obtain an IC₅₀ value of the compound in inhibition on cell proliferation, which are shown in Table 1.

**Table 1 IC₅₀ data of the compounds of the present invention demonstrating inhibition on OVCAR-3 cell proliferation**

| Serial number of example | IC₅₀ (nM) | Serial number of example | IC₅₀ (nM) | Serial number of example | IC₅₀ (nM) |
|---|---|---|---|---|---|
| Control compound AMG 650 | 64.5 | **14** | 8.4 | **22** | 33.2 |
| **1** | 0.75 | **15** | 18.4 | **23** | 3.6 |
| **2** | 4.3 | **16** | 36.7 | **24** | 13.4 |
| **3** | 11.2 | **17** | 6.6 | **25** | 33.6 |
| **6** | 9.1 | **18** | 3.3 | **27** | 1.96 |
| **7** | 11 | **20** | 20.3 | **28** | 6.8 |
| **13** | 5.5 | **21** | 16.6 | | |

Conclusion: the compounds of the present invention have a relatively good inhibitory effect on OVCAR-3 cell proliferation IC₅₀<50 nM.

Note: the structure of AMG 650 (prepared according to Example 4 of the published patent WO2020132648A1) was as follows:

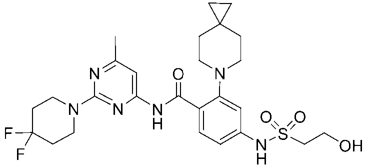

### Test Example 2. Determination of inhibition of the compounds of the present invention on HT-29 cell proliferation

Influences of the compounds of the present invention on HT-29 cell proliferation were determined by the following method. HT-29 cells (containing TP53 R273H mutation) were purchased from the Cell Bank of Shanghai Institute of Biochemistry and Cell Biology, Chinese Academy of Sciences, and cultured in a McCOY's 5A culture medium containing 10% fetal bovine serum, 100 U penicillin and 100 µg/mL streptomycin. A cell viability was determined by a CellTiter-Glo^{®} Luminescent Cell Viability Assay kit (Promega, article number G7573).

An experimental method was operated according to steps in the instruction of the kit, which was briefly described as follows: a test compound was dissolved in DMSO first to prepare a 10 mM stock solution, then the stock solution was diluted with the above McCOY's 5A culture medium to prepare a test sample, and a final concentration of the compound was in a range of 1000 nM-0.015 nM. Cells in a logarithmic growth phase were inoculated into a 96-well cell culture plate at a density of 1000 cells per well, cultured in a 5% CO₂ incubator at 37°C overnight, and then the test compound was added to continuously culture for 120 hours. After the culture was ended, 50 µL of CellTiter-Glo detection solution was added to each well, the plates were shaken for 5 minutes, and then allowed to stand for 10 minutes, and subsequently, a luminous value of each well of sample was read by a microplate reader in a Luminescence mode. By comparing with a numerical value of a control group (0.3% DMSO), a percentage inhibition rate of the compound at each concentration was calculated, and then nonlinear regression analysis was carried out based on concentration logarithm-inhibition rate of the compound in GraphPad Prism 5 software, so as to obtain an IC₅₀ value of the compound in inhibition on cell proliferation, which are shown in Table 2.

**Table 2 IC₅₀ data of the compounds of the present invention demonstrating inhibition of HT-29 cell proliferation**

| Serial number of example | IC₅₀ (nM) | Serial number of example | IC₅₀ (nM) | Serial number of example | IC₅₀ (nM) |
|---|---|---|---|---|---|
| Control compound AMG 650 | 61.7 | **14** | 9.2 | **28** | 18.9 |
| **1** | 2.4 | **15** | 23.6 | | |
| **2** | 3.9 | **16** | 35.7 | | |
| **3** | 6.6 | **18** | 7.7 | | |
| **6** | 10.7 | **21** | 13.8 | | |
| **7** | 7.8 | **23** | 5.8 | | |
| **13** | 7.3 | **24** | 4.1 | | |

Conclusion: the compounds of the present invention have a relatively good inhibitory effect on HT-29 cell proliferation, IC₅₀<50 nM.

### Test Example 3. Testing of inhibition of the compounds of the present invention on KIF18A enzyme activity

Inhibition degrees of the compounds of the present invention on recombinant human KIF18A enzyme activity in vitro were determined by the following method. An ADP-Glo^{™} Kinase Assay kit (article number V9102) from Promega Company was used in this method. Detailed experimental operation can be referred to the instruction of the kit.

An experimental flow was briefly described as follows: a test compound was dissolved in DMSO first to prepare a stock solution, then the stock solution was gradiently diluted with a reaction buffer A (15 mM Tris, pH 7.5, 10 mM MgCl₂, 0.01% Pluronic F-68), and a final concentration of the test compound in the reaction system was in a range of 10000 nM-0.15 nM; and a KIF18A protein and ATP working solution was prepared by using a reaction buffer B (15 mM Tris, pH 7.5, 10 mM MgCl₂, 0.01% Pluronic F-68, 37.5 µg/ml tubulin, 1.25 µM paclitaxel). The reaction was carried out in a 384-well microplate. The test compound and the recombinant human KIF18A protein (final concentration of 100 nM, entrusted to GenScript for expression) were added into the wells and incubated at room temperature for 20 minutes, and subsequently, ATP solution (component V915A from the ADP-Glo^{™} Kinase Assay kit, final concentration of 60 µM) was added to the reaction solution and the obtained system was incubated at room temperature for 20 minutes. Subsequently, 5 µL of ADP-Glo Reagent was added to the reaction system and the obtained system was incubated at room temperature for 50 minutes. Then, 10 µL of Kinase Detection Reagent was added to the reaction system, and the obtained system was incubated at room temperature for 30 minutes. After the incubation was ended, a chemiluminiscence intensity value of each well was determined by a microplate reader in a Luminescence mode. By comparing with a luminous intensity value of a control group (0.1% DMSO), a percentage inhibition rate of the compound at each concentration was calculated, and nonlinear regression analysis was carried out based on concentration logarithm value-inhibition rate of the compound in GraphPad Prism 5 software, so as to obtain an IC₅₀ value of the compound, which are shown in Table 3.

**Table 3 IC₅₀ data of the compounds of the present invention demonstrating inhibition on KIF18A enzyme activity**

| Serial number of example | IC₅₀ (nM) | Serial number of example | IC₅₀ (nM) | Serial number of example | IC₅₀ (nM) |
|---|---|---|---|---|---|
| Control compound AMG 650 | 173 | **11** | 263 | **24** | 85.7 |
| **1** | 52 | **12** | 173 | **25** | 198 |
| **2** | 142 | **13** | 291 | **26** | 256 |
| **3** | 72.5 | **14** | 184 | **27** | 109 |
| **4** | 212 | **16** | 209 | **28** | 142 |
| **5** | 134.3 | **17** | 280 | **29** | 316 |
| **6** | 60.9 | **20** | 197 | | |
| **7** | 157 | **21** | 229 | | |
| **8** | 90.6 | **22** | 98.5 | | |
| **10** | 267 | **23** | 123 | | |

Conclusion: the compounds of the present invention have a significant inhibitory effect on KIF18A enzyme activity, IC₅₀<500 nM.

### Test Example 4. Pharmacokinetic testing of the compounds of the present invention in mice

1. Experimental purpose
   ICR mice were taken as test animals, and intragastrically administered with a control compound AMG650 and compounds 1, 18 and 29 of the present invention, and then drug concentrations in plasma at different moments were determined by an LC/MS/MS method, so as to study pharmacokinetic characteristics of the compounds of the present invention in the mice.
2. Experimental scheme
   2.1 Experimental drugs and animals
      Control compound AMG650, and Compounds 1, 18 and 29.
      ICR mice, male, 27.3-30.3 g, purchased from Vital River Laboratory Animal Technology Co., Ltd.
   2.2 Preparation of drugs
      A proper amount of to-be-tested compound was weighed, proper amounts of DMA (N,N-dimethylacetamide), CrEL (polyoxyethylene 35 castor oil) and 5% GS (5% glucose injection) were added in sequence, and the obtained system was evenly mixed by ultrasonic vortex to prepare a 1 mg/mL preparation, wherein DMA, CrEL and 5% GS=10: 10: 80 (v: v: v).
   2.3 Administration
      ICR mice in each to-be-tested compound injection group (9 mice in each group) were fasted overnight and then intragastrically administered with the compound (PO, an administration dosage of the compound was 10 mg/kg and an administration volume of the compound was 10 mL/kg), and were fed 4 hours after administration.
3. Operation
   0.25 hours, 0.5 hours, 1 hour, 2 hours, 4 hours, 8 hours, 10 hours and 24 hours before and after administration, about 0.1 µL of blood was collected through an orbit, and a complete blood sample was placed in an EDTA-K2-containing anticoagulation tube. The collected blood sample was placed on ice, and plasma was centrifugally separated (centrifugation conditions: 1500 g, 10 minutes). The collected plasma was stored at -40 ~ -20°C before analysis.
   A content of the to-be-tested compound in the plasma of the mice after intragastric administration was determined by LC-MS/MS.
4. Results of pharmacokinetic parameters
   Pharmacokinetic parameters of to-be-tested compounds are shown in Table 4 below.

**Table 4 Pharmacokinetic parameters of to-be-tested compounds in mice**

| Serial number of compound | Pharmacokinetic experiment | | | |
|---|---|---|---|---|
| | Administration mode (Administration dosage) | Plasma concentration Cmax (ng/mL) | Area under the curve AUC_{0-∞} (ng·h/mL) | Half-life period T1/2 (h) |
| Control compound AMG650 | Oral administration (10 mg/mL) | 3050 | 52500 | 9.64 |
| Example 1 | Oral administration (10 mg/mL) | 12417 | 95245 | 7.36 |
| Example 18 | Oral administration (10 mg/mL) | 3030 | 45900 | 5.2 |
| Example 29 | Oral administration (10 mg/mL) | 6960 | 25881 | 1.24 |

Conclusion: the compounds 1, 18 and 29 of the present invention have both a high plasma concentration and a large area under the curve, and have good pharmacokinetic properties.

### Test Example 5. Pharmacodynamic testing of Compound 1 of the present invention in mice

### 1. Experimental purpose

An anti-tumor effect of Compound 1 of the present invention in a BALB/c nude mouse animal model with subcutaneous OVCAR-3 human ovarian adenocarcinoma tumor fragment xenograft was evaluated.

### 2. Experimental animals

BALB/c nude mice, female, 6-7 weeks old, purchased from Jiangsu Gempharmatech Biotechnology Co., Ltd.

### 3. Preparation of test compounds

Mice in a solvent control group were given DMA: CrEL: 5% GS=10: 10: 80 (v: v: v).

AMG650: a proper amount of AMG-650 was weighed, proper amounts of DMA, CrEL and 5% GS were added in sequence, and the obtained system was evenly mixed by ultrasonic vortex to prepare 0.8 mg/mL and 0.4 mg/mL preparations respectively, wherein DMA, CrEL and 5% GS=10: 10: 80 (v: v: v).

Compound 1: a proper amount of Compound 1 was weighed, proper amounts of DMA, CrEL and 5% GS were added in sequence, and the obtained system was evenly mixed by ultrasonic vortex to prepare 0.8 mg/mL and 0.4 mg/mL preparations respectively, wherein DMA, CrEL and 5% GS=10: 10: 80 (v: v: v).

### 4. Acquisition of inoculation tumor fragment

An appropriate OVCAR-3 tumor-bearing animal was selected, a 1 mm×1 mm×1 mm OVCAR-3 tumor fragment was acquired aseptically and placed into normal saline for later use, which was used for subcutaneous tumor inoculation in a right side of back of the BALB/c nude mice.

### 5. Animal inoculation and grouping

About 1 mm×1 mm×1 mm OVCAR-3 tumor fragment was subcutaneously inoculated in the right side of back of the female BALB/c nude mice. When an average tumor volume reached about 100-250 mm³, the mice were randomly grouped according to the tumor size, with 6 mice in each group.

### 6. Administration and observation to animals

Animals in each group were given the test compound once per day according to an animal weight at a fixed time every day by oral administration (po), the animals started to be administered with the test compound for the first time on the day of grouping for 22 consecutive days, and the animal weight was recorded every day.
The 1^{st} group (G1) was a solvent control group;
The 2^{nd} group (G2) and the 3^{rd} group (G3) were orally administered with AMG-650 at administration dosages of 8 mg/kg and 4 mg/kg respectively once per day (QD); and
The 4^{th} group (G4) and the 5^{th} group (G5) were orally administered with Compound 1 at administration dosages of 8 mg/kg and 4 mg/kg respectively once per day (QD).

Tumor formation at an inoculation site of the animals in each group was observed, and a tumor volume was measured twice per week, and calculated according to the following formula:
a tumor volume (TV), a relative tumor volume (RTV), a relative tumor proliferation rate (T/C) and a relative tumor inhibition rate (TGI) were calculated according to the following formulas:
   (1) TV (tumor volume)= 1/2×a×b², wherein a and b respectively represented a tumor length and a tumor width;
   (2) RTV (relative tumor volume)= Vₜ/V₀, wherein V₀ was a tumor volume measured at the time of grouping, and Vₜ was a tumor volume in each measurement;
   (3) T/C (%)= T_{RTV}/ C_{RTV}×100%, wherein T_{RTV} was RTV of a treatment group, and C_{RTV} was RTV of a solvent control group; and
   (4) TGI% =(1-T/C) × 100%, wherein T and C were relative tumor volumes of the treatment group and the solvent control group at a specific time point respectively.

### 7. Results

**Table 5 Efficacy analysis table of test compounds across dose groups in OVCAR-3 human ovarian adenocarcinoma tumor fragment xenograft model**

| Group | 16 days after administration | | |
|---|---|---|---|
| | Tumor volume (x±S) | Relative tumor volume (x±S) | TGI% |
| 1^{st} group Solvent control group | 620±80 | 3.27±0.28 | - |
| 2^{nd} group AMG650 (8 mg/kg) | 243±35 | 1.29±0.16 | 60.5% |
| 3^{rd} group AMG650 (4 mg/kg) | 511±49 | 2.77±0.26 | 15.3% |
| 4^{th} group Compound 1 (8 mg/kg) | 31±8 | 0.16±0.03 | 95.2% |
| 5^{th} group Compound 1 (4 mg/kg) | 120±27 | 0.61±0.11 | 81.3% |

| | | | |
|---|---|---|---|
| Note: the data were expressed by "mean value ± standard error". | | | |

Conclusion: under the experimental conditions specified in this study, Compound 1 of the present invention demonstrated significantly enhanced inhibition of tumor growth at both 8 mg/kg and 4 mg/kg doses compared to the solvent control and AMG650 in a BALB/c nude mouse model bearing subcutaneous OVCAR-3 human ovarian adenocarcinoma tumor fragment xenografts.

Unless otherwise defined, all terms used in the present invention have the meanings commonly understood by those skilled in the art.

The embodiments described in the present invention are only for illustrative purposes, and are not intended to limit the scope of protection of the present invention, and those skilled in the art can make various other substitutions, changes and improvements within the scope of the present invention. Therefore, the present invention is not limited to the embodiments above, but only defined by the claims.

## Claims

1. A compound as shown by general formula (I), or a stereoisomer thereof, a tautomer thereof or a pharmaceutically acceptable salt thereof: 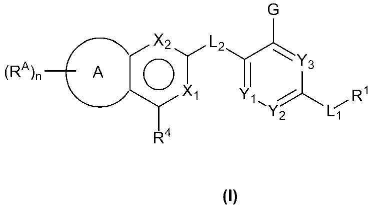 wherein:
ring A is selected from 5-7 membered heterocyclyl or 5-7 membered heteroaryl;
G is selected from 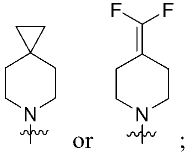
X₁ and X₂ are each independently selected from CR^{a} or an N atom;
Y₁, Y₂ and Y₃ are each independently selected from CR^{b} or an N atom, and at most two of Y₁, Y₂ and Y₃ are N atom at the same time;
R^{a} and R⁶ are each independently selected from a hydrogen atom, halogen, hydroxyl, cyano, alkyl or alkoxy, wherein the alkyl or the alkoxy is optionally further substituted by one or more substituents selected from halogen, hydroxyl, cyano, alkyl or alkoxy;
R^{A} is the same or different, and is each independently selected from a hydrogen atom, halogen, hydroxyl, cyano, alkyl or alkoxy, wherein the alkyl or the alkoxy is optionally further substituted by one or more substituents selected from halogen, hydroxyl, cyano, alkyl or alkoxy;
or, two R^{A} form a C(O) together with the same carbon atom to which the two R^{A} are attached;
L₁ is selected from a bond or C₁-C₆ alkylene, wherein the alkylene is optionally further substituted by one or more substituents selected from halogen, hydroxyl, cyano or alkoxy, and one or more methylene groups of the alkylene are optionally substituted by one or more O, S(O)ᵣ, C(O) or NR^{c};
R^{c} is selected from a hydrogen atom or alkyl;
L₂ is selected from 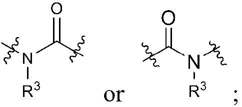
R³ is each independently selected from a hydrogen atom or alkyl, wherein the alkyl is optionally further substituted by one or more substituents selected from halogen, hydroxyl, cyano or alkoxy; and R³ is preferably a hydrogen atom;
R¹ is selected from a hydrogen atom, cyano, halogen, alkyl, hydroxyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁴ is selected from cyano, halogen, alkyl, alkenyl, alkynyl, hydroxyl, cycloalkyl, heterocyclyl, aryl, heteroaryl, -OR⁵, -C(O)R⁵, -C(O)OR⁵, -NHC(O)R⁵, -NHC(O)OR⁵, -NR⁶R⁷, -C(O)NR⁶R⁷, -CH₂NHC(O)OR⁵, -CH₂NR⁶R⁷ or -S(O)ᵣR⁵, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁵ is each independently selected from a hydrogen atom, alkyl, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, cyano, alkyl, alkoxy, halogenated alkyl, halogenated alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁶ and R⁷ are each independently selected from a hydrogen atom, hydroxyl, halogen, alkyl, alkoxy, cycloalkyl, heterocyclyl, aryl or heteroaryl, wherein the alkyl, the alkoxy, the cycloalkyl, the heterocyclyl, the aryl or the heteroaryl is optionally further substituted by one or more substituents selected from hydroxyl, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
or, R⁶ and R⁷ form a 4-8 membered heterocyclyl together with the atoms to which the R⁶ and the R⁷ are attached, wherein the 4-8 membered heterocyclyl contains one or more N, O or S(O)ᵣ, and the 4-8 membered heterocyclyl is optionally further substituted by one or more substituents selected from hydroxyl, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰ or -NR⁹C(O)R¹⁰;
R⁸, R⁹ and R¹⁰ are each independently selected from a hydrogen atom, alkyl, amino, cycloalkyl, heterocyclyl, aryl or heteroaryl, wherein the alkyl, the cycloalkyl, the heterocyclyl, the aryl or the heteroaryl is optionally further substituted by one or more substituents selected from hydroxyl, halogen, nitro, amino, cyano, alkyl, alkoxy, cycloalkyl, heterocyclyl, aryl, heteroaryl, carboxyl or carboxylate group.
n is 0, 1, 2, 3 or 4; and
r is each independently 0, 1 or 2.

2. The compound, or the stereoisomer thereof, the tautomer thereof or the pharmaceutically acceptable salt thereof according to claim 1, which is a compound as shown by formula (II) or (III), or a stereoisomer thereof, a tautomer thereof or a pharmaceutically acceptable salt thereof: 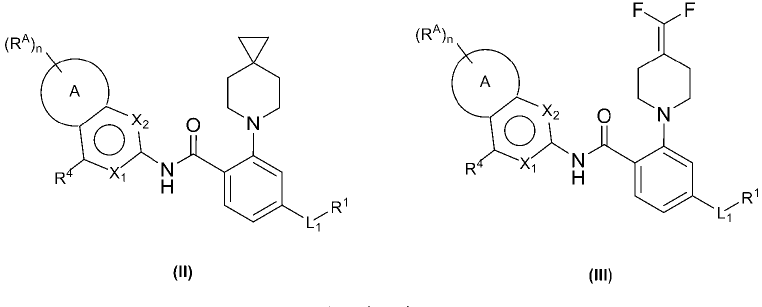 wherein, definitions of ring A, X₁, X₂, R^{A}, R¹, R⁴, L₁ and n are as described in claim 1.

3. The compound, or the stereoisomer thereof, the tautomer thereof or the pharmaceutically acceptable salt thereof according to claim 1 or 2, wherein:
L₁ is selected from a bond or C₁-C₆ alkylene, wherein the alkylene is optionally further substituted by one or more hydroxyl groups, and one or more methylene groups of the alkylene are optionally substituted by one or more O, S(O)ᵣ, C(O) or NR^{c};
r is 2; and
R^{c} is selected from a hydrogen atom or methyl.

4. The compound, or the stereoisomer thereof, the tautomer thereof or the pharmaceutically acceptable salt thereof according to claim 3, wherein L₁ is selected from a bond, -NHSO₂CH₂CH₂-, -SO₂NHCH₂CH₂-, -SO₂-, -CH₂SO₂-, -NHSO₂-, -SO₂NH-, -NHC(CH₃)₂CH₂-, -C(O)NHCH₂CH₂-, -C(O)NHC(CH₃)₂CH₂-, -C(O)N(CH₃)CH₂CH₂-, -CH(CH₃)(OH)CH₂-, -NHSO₂CH(CH₃)CH₂-, -SO₂NHC(CH₃)₂CH₂-, -C(O)NH-, -NHCH₂CH₂- or -CH₂SO₂CH₂CH₂-.

5. The compound, or the stereoisomer thereof, the tautomer thereof or the pharmaceutically acceptable salt thereof according to claim 1 or 2, wherein R¹ is selected from a hydrogen atom, hydroxyl, alkyl, heterocyclyl, cycloalkyl or heteroaryl, wherein the alkyl, the heterocyclyl, the cycloalkyl or the heteroaryl is optionally further substituted by one or more substituents selected from hydroxyl or alkyl.

6. The compound, or the stereoisomer thereof, the tautomer thereof or the pharmaceutically acceptable salt thereof according to claim 1 or 2, wherein 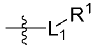 is 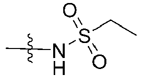 or

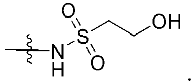

7. The compound, or the stereoisomer thereof, the tautomer thereof or the pharmaceutically acceptable salt thereof according to claim 1 or 2, wherein 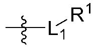 is

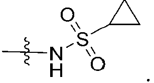

8. The compound, or the stereoisomer thereof, the tautomer thereof or the pharmaceutically acceptable salt thereof according to claim 1 or 2, wherein X₁ and X₂ are each independently selected from CH.

9. The compound, or the stereoisomer thereof, the tautomer thereof or the pharmaceutically acceptable salt thereof according to claim 1 or 2, wherein ring A is selected from:

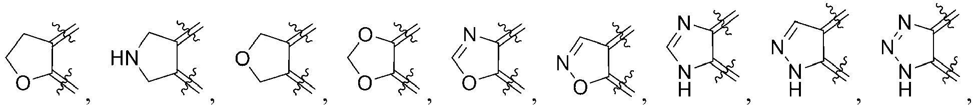

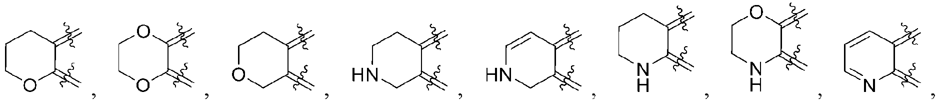

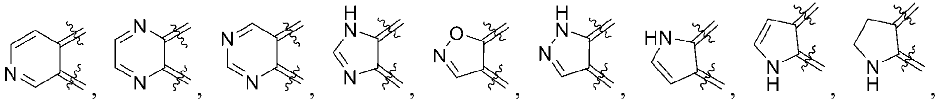

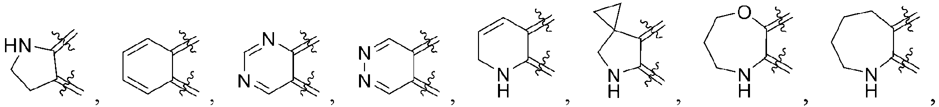

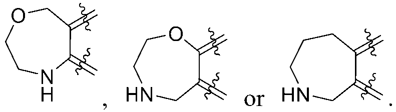

10. The compound, or the stereoisomer thereof, the tautomer thereof or the pharmaceutically acceptable salt thereof according to claim 1 or 2, wherein ring A is selected from

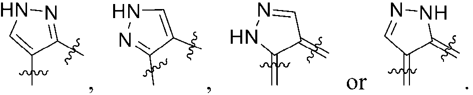

11. The compound, or the stereoisomer thereof, the tautomer thereof or the pharmaceutically acceptable salt thereof according to claim 1 or 2, wherein:
R^{A} is each independently selected from a hydrogen atom or methyl;
or, two R^{A} form a C(O) together with the same carbon atom to which the two R^{A} are attached.

12. The compound, or the stereoisomer thereof, the tautomer thereof or the pharmaceutically acceptable salt thereof according to claim 1 or 2, wherein:
R⁴ is each independently selected from 3-10 membered heterocyclyl or C₃-C₁₀ cycloalkyl, wherein the heterocyclyl or the cycloalkyl is optionally further substituted by one or more hydroxyl groups or halogens.

13. The compound, or the stereoisomer thereof, the tautomer thereof or the pharmaceutically acceptable salt thereof according to claim 1 or 2, wherein:
R⁴ is each independently selected from C₂-C₆ alkenyl or C₁-C₆ alkyl, wherein the alkenyl or the alkyl is optionally further substituted by one or more hydroxyl groups or halogens.

14. The compound, or the stereoisomer thereof, the tautomer thereof or the pharmaceutically acceptable salt thereof according to claim 12, wherein:
R⁴ is

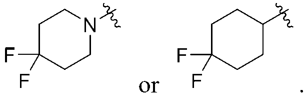

15. The compound, or the stereoisomer thereof, the tautomer thereof or the pharmaceutically acceptable salt thereof according to claim 1 or 2, wherein R⁴ is

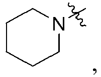

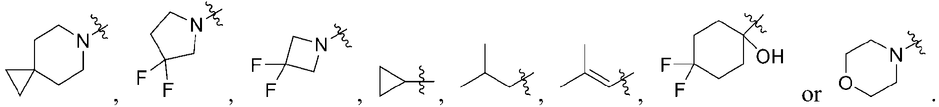

16. The compound, or the stereoisomer thereof, the tautomer thereof or the pharmaceutically acceptable salt thereof according to any one of claims 1 to 15, wherein the compound is: 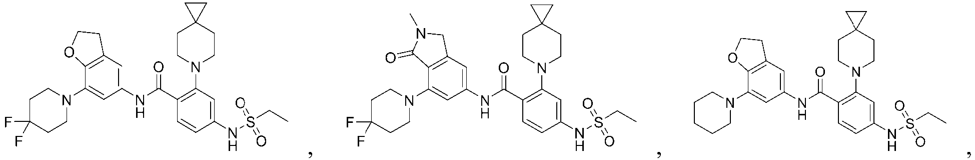 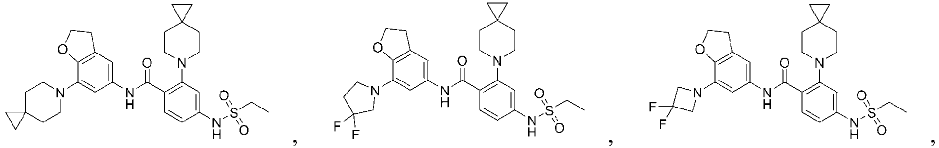 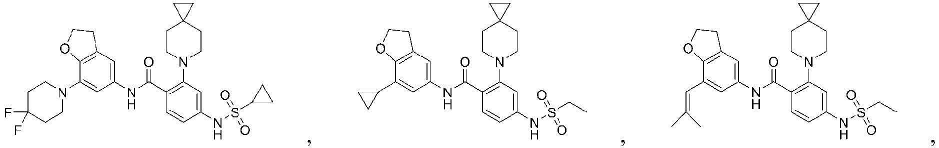 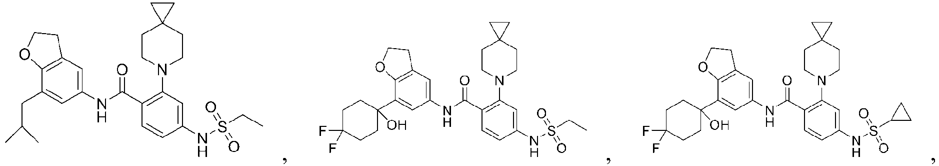 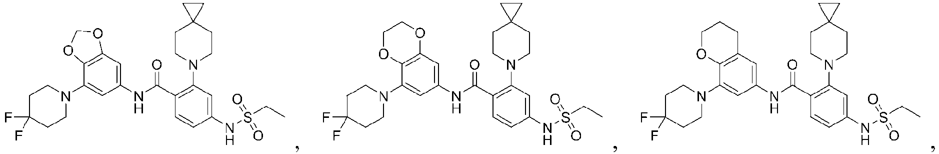 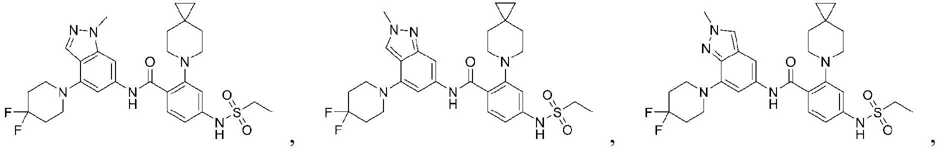 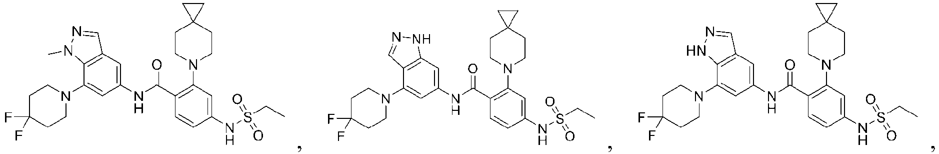 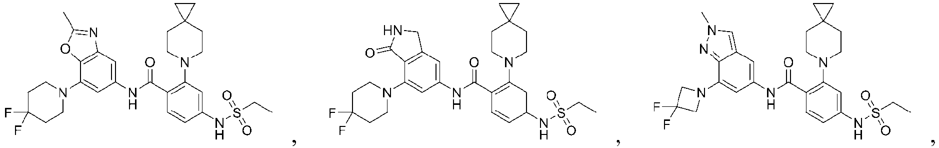 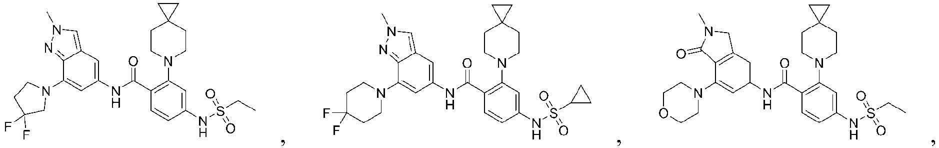 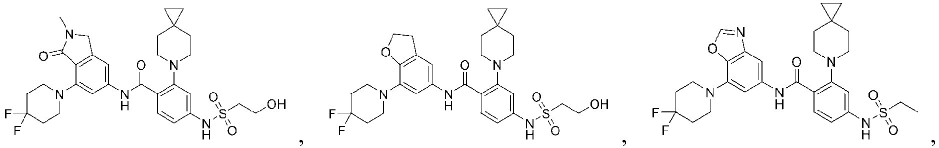 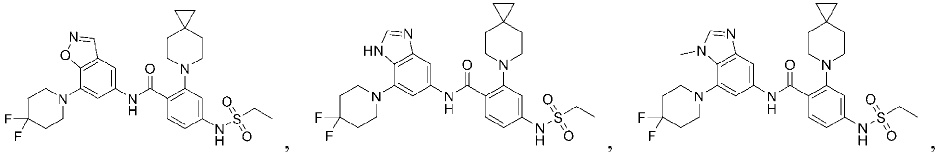 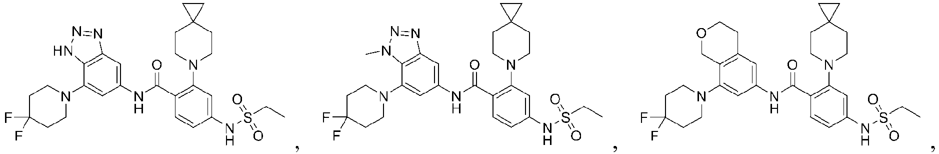 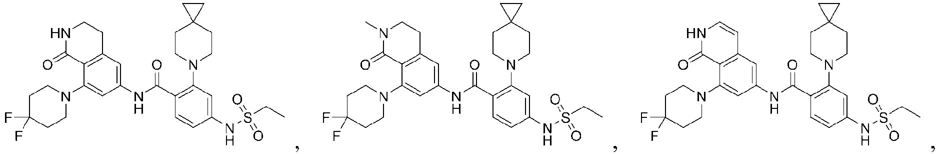 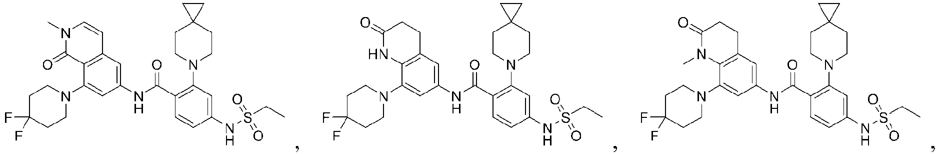 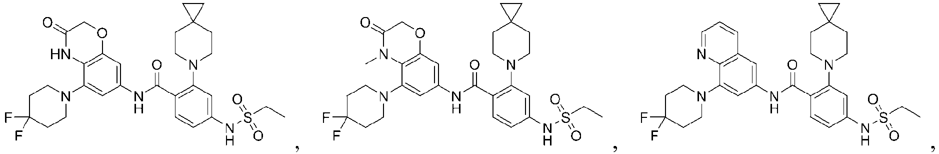 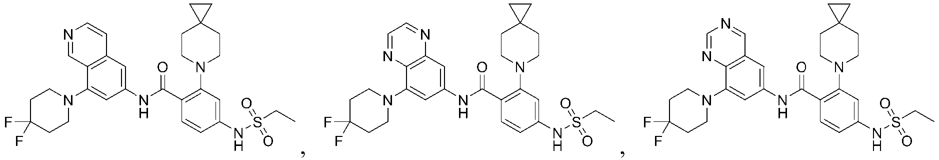 or

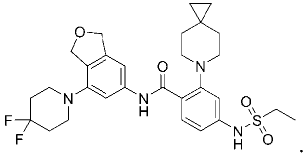

17. A pharmaceutical composition, wherein the pharmaceutical composition comprises an effective amount of the compound, or the stereoisomer thereof, the tautomer thereof or the pharmaceutically acceptable salt thereof according to any one of claims 1 to 16, and a pharmaceutically acceptable carrier, an excipient or a combination of the two.

18. Use of the compound, or the stereoisomer thereof, the tautomer thereof or the pharmaceutically acceptable salt thereof according to any one of claims 1 to 16, or the pharmaceutical composition according to claim 17 in the preparation of a KIF18A inhibitor.

19. Use of the compound, or the stereoisomer thereof, the tautomer thereof or the pharmaceutically acceptable salt thereof according to any one of claims 1 to 16, or the pharmaceutical composition according to claim 17 in the preparation of a medicament for treating a KIF18A-mediated disease, wherein the KIF18A-mediated disease is preferably cancer.

20. The use according to claim 19, wherein the cancer is selected from a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, a colorectal cancer or a lung adenocarcinoma.

21. Use of the compound, or the stereoisomer thereof, the tautomer thereof or the pharmaceutically acceptable salt thereof according to any one of claims 1 to 16, or the pharmaceutical composition according to claim 17 in the preparation of a medicament for treating cancer.

22. The use according to claim 21, wherein the cancer is selected from a hepatocellular carcinoma, a glioblastoma, a colon cancer, a breast cancer, a lung cancer, a cholangiocarcinoma, a pancreatic cancer, a prostate cancer, a bladder cancer, a head cancer, a neck cancer, a cervical cancer, an ovarian cancer, a synovial sarcoma, a rhabdomyosarcoma, a colorectal cancer or a lung adenocarcinoma.
